# EUROPEAN PATENT APPLICATION

(11) **EP 1 813 948 A1**
(43) Date of publication of application: **01.08.2007**
(21) Application number: 05800167.8
(22) Date of filing: 01.11.2005
(51) Int. Cl.: G01N 33/543, G01N 27/447, C12Q 1/68

(54) **METHOD OF DETECTING TARGET SUBSTANCES**

(30) Priority: 01.11.2004 JP 2004318395
(71) Applicant: Tokyo Metropolitan Organization for Medical Research, Shinjuku-ku, Tokyo 163-8001 (JP); Synthera Technologies Co., Ltd., Tokyo 113-0021 (JP)
(72) Inventor: SHIBASAKI, Futoshi, 1750094 (JP); SAKATA, Kazuhiko, 2970012 (JP)
(74) Representative: Denison, Christopher Marcus
(86) International application number: PCT/JP2005/020402
(87) International publication number: WO 2006/049289

(57) **Abstract**

The present invention provides a method for identifying and detecting multiple types of target substances contained in a specimen, comprising the steps of: contacting the target substances immobilized and/or nonimmobilized on a support with labeled binders capable of identifying corresponding types of target substances so as to form complexes between the target substances and the binders; and detecting the labels in the binders forming the complexes.

SEQUENCE LISTING

<110> TOKYO METROPOLITAN ORGANIZATION FOR MEDICAL RESEARCH
SYNTHERA TECHNOLOGIES CO., LTD.

<120> METHOD OF DETECTING TARGET SUBSTANCES

<130> G07-0031

<140> PCT/JP2005/020402
<141> 2005-11-01

<150> JP 2004-318395
<151> 2004-11-01

<160> 14

<170> PatentIn version 3.3

<210> 1
<211> 38
<212> DNA
<213> Artificial

<220>
<223> primer

<400> 1
cggaattcgc ggacgaggag aagctgccgc ccggctgg 38

<210> 2
<211> 24
<212> DNA
<213> Artificial

<220>
<223> primer

<400> 2
gctggcgtta gtgatgtggt tgaa 24

<210> 3

<211>  18
<212>  DNA
<213>  Artificial

<220>
<223>  primer

<400>  3
ctggctgtgc ttcaccag                                                    18


<210>  4
<211>  18
<212>  DNA
<213>  Artificial

<220>
<223>  primer

<400>  4
cttactggct tatcgaaa                                                    18


<210>  5
<211>  18
<212>  DNA
<213>  Artificial

<220>
<223>  primer

<400>  5
cattttatta ggaaagga                                                    18


<210>  6
<211>  18
<212>  DNA
<213>  Artificial

<220>
<223>  primer

<400>  6

cgcgcttaat gcgccgct 18

<210> 7
<211> 18
<212> DNA
<213> Artificial

<220>
<223> primer

<400> 7
cttactggct tatcgaaa 18

<210> 8
<211> 38
<212> DNA
<213> Artificial

<220>
<223> primer

<400> 8
ggcaagccac gtttggtgca gtcgaggctg atcagcga 38

<210> 9
<211> 38
<212> DNA
<213> Artificial

<220>
<223> primer

<400> 9
ggcaagccac gtttggtgtc ctcattttat taggaaag 38

<210> 10
<211> 38
<212> DNA
<213> Artificial

<220>
<223>  primer

<400>  10
ggcaagccac gtttggtgag catgcctgct attgtctt                                38

<210>  11
<211>  38
<212>  DNA
<213>  Artificial

<220>
<223>  primer

<400>  11
ggcaagccac gtttggtgcc cgccgcgctt aatgcgcc                                38

<210>  12
<211>  38
<212>  DNA
<213>  Artificial

<220>
<223>  primer

<400>  12
ggcaagccac gtttggtgaa agccggcgaa cgtggcga                                38

<210>  13
<211>  25
<212>  DNA
<213>  Artificial

<220>
<223>  primer

<400>  13
tgcatctaga gggccctatt ctata                                              25

<210>  14

<211>  18
<212>  DNA
<213>  Artificial

<220>
<223>  primer

<400>  14
ggcaagccac gtttggtg                                                    18

## Description

### FIELD OF THE INVENTION

The present invention relates to a method for detecting a target substance using a reaction between the target substance and a substance that can specifically bind thereto (for example, an antigen-antibody reaction). More particularly, the present invention relates to a method for identifying and detecting multiple types of target substances contained in a specimen.

### BACKGROUND OF THE INVENTION

Establishment of the monoclonal antibody preparation technique has enabled the acquisition of an antibody that can specifically react with a certain antigen. Since then, development and improvement of an assay system for detecting the antigen using specificity of antigen-antibody reaction have become essential in the fields of research, clinic and else. Generally, in such an assay system, an antibody that reacts with the antigen is labeled with a certain label so that this label can be detected after the reaction for detecting the antigen. Furthermore, in order to further enhance the detection sensitivity, various studies about, for example, various types of labels and methods for detecting the labels have also been conducted continually. For example, other than the methods using an avidin-biotin system, a fluorescent dye or a radioisotope, examples of such methods with enhanced detection sensitivity include Immuno-PCR method (see, for example, T. Sano et al., Science, vol. 258, 120-122 (1992)), Double Determinant Immuno-PCR method (see, for example, Kozo Imai, Asako Suzuki and Yuji Hinoda, "Detection of Small Amount of Antigen Using Immuno-PCR" Protein, Nucleic Acid and Enzyme, Yodosha, 1996, Vol.41, No.5, p.614-617) and a detection method using a western blotting with an oligonucleotide and a DNA dendrimer (see, for example, Japanese Patent Application Publication No. 2001-503517).

### DISCLOSURE OF THE INVENTION

All of the detection methods mentioned above, however, can detect only one particular type among multiple types of target substances (antigens) contained in a specimen, and no method has been capable of identifying and detecting two or more particular types of target substances simultaneously so far. Thus, when all of the two or more types of target substances are to be detected, they have to be detected in a separate detection system for each of their types, which is problematic in terms of detection efficiency including time and labor required for detection. In fact, recently, needs for detecting a wide variety of target substances (biomarkers, causative substances, pathogens, etc.) from a specimen is growing increasingly. Examples in the clinical examination field include diagnosis of cancer, drug efficacy evaluations of anticancer drugs, diagnosis of cerebral stroke, diagnosis of myocardial infarction and identification of pathogens in infectious diseases, and examples in the food and environment field include identification of food contaminants such as pesticide residues and bacteria and identification of environmental pollutants such as dioxin and polychlorinated biphenyl (PCB). Thus, a method capable of detecting these substances by a single round of detection treatment has been desired.

Thus, the problem to be solved by the present invention is to provide a method for detecting target substances, which is capable of identifying and detecting multiple types of target substances contained in a specimen at once.

The present inventors have devoted themselves to studies for solving the problem above. As a result, they found that by providing substances (binders) that specifically bind to each of two or more types of target substances to be detected among other multiple types of target substances contained in a specimen, by labeling each of the binders to identify the corresponding types of the target substances and by allowing formations of complexes between the target substances and the binders via specific binding reaction therebetween, simultaneous and clear distinction of the labels unique to the respective complexes (namely simultaneous multiple detection) can be realized in the subsequent detection stage, thereby accomplishing the present invention.

Thus, the present invention is as follows.
(1) A method for identifying and detecting multiple types of target substances contained in a specimen, comprising the steps of:
   (i) contacting target substances immobilized and/or nonimmobilized on a support with binders that are labeled for identifying the corresponding types of target substances so as to allow formation of complexes between the target substances and the binders (hereinafter, the step of forming target substance-binder complexes); and
   (ii) detecting the labels of the binders forming the complexes (hereinafter, the step of detecting the labels).

   According to the method of the present invention, the label moiety of the binder may be attached to the binder via at least an adapter moiety. The adapter moiety may be, for example, one selected from the group consisting of protein G, protein A, protein L and a fusion protein of protein A and protein G.
   According to the method of the present invention, the binder may be, for example, one that forms a conjugate (oligonucleotide conjugate) with an oligonucleotide chain as a label substance. The labeling treatment mentioned above may include, for example, the following treatments (i)-(ix) that are performed on the oligonucleotide chain mentioned above.
   (i) Treatment of providing a site cleavable with a restriction enzyme.
   (ii) Treatment of providing a region amplifiable by PCR.
   (iii) Treatment of incorporating a radioisotope element.
   (iv) Treatment of binding a fluorescent dye.
   (v) Treatment of binding an enzyme.
   (vi) Treatment of providing a site cleavable by light irradiation.
   (vii) Treatment of providing a site cleavable with active oxygen.
   (viii) Treatment of binding a labeled dendrimer.
   (ix) Treatment of providing a difference by at least one base type.

   Moreover, the step of defecting the label may include, for example, a step of detecting the PCR-amplified product by electrophoresis.
   According to the method of the present invention, the binder may include, for example, one that forms a conjugate (oligopeptide conjugate) with an oligopeptide chain as a label substance. The labeling treatment may include, for example, the following treatments (i)-(vii) that are performed on the oligopeptide chain mentioned above.
   (i) Treatment of providing a site cleavable with protease.
   (ii) Treatment of incorporating a radioisotope element.
   (iii) Treatment of binding a fluorescent dye.
   (iv) Treatment of binding an enzyme.
   (v) Treatment of providing a site cleavable by light irradiation.
   (vi) Treatment of providing a site cleavable with active oxygen.
   (vii) Treatment of binding a labeled dendrimer.
      According to the method of the present invention, the target substance may be, for example, an antigen, while the binder may be, for example, an antibody.
(2) A kit for identifying and detecting target substances, comprising multiple types of labeled binders to allow identification of corresponding types of target substances.

### BRIEF DESCRIPTION OF THE DRAWINGS

Figure 1 is a schematic flow diagram showing an example of identifying and detecting an antigen using a restriction enzyme reaction.
Figure 2 is a schematic flow diagram showing an example of identifying and detecting an antigen using Immuno-PCR method.
Figure 3A is a schematic view showing an example of identifying and detecting an antigen using free radical.
Figure 3B is a schematic view showing an example of identifying and detecting an antigen using an antibody conjugated with an oligopeptide nucleic acid chain.
Figure 4 is a picture showing bands after agarose gel electrophoresis in an assay of the conjugated antibody in Example 1.
Figure 5 is a picture showing a band after agarose gel electrophoresis in identification and detection using the conjugated antibody in Example 1.
Figure 6 is a chart showing the results of measurement with cosmo-i SV1210 in Example 2. In the chart, the numerals above the peaks are the values representing the peak areas (oligonucleotide concentrations).
Figure 7 is a schematic view showing an example of identifying and detecting an antigen using an antibody conjugated with an oligopeptide nucleic acid chain via an adapter moiety.
Figure 8 is a chart showing the results upon GeneScan analysis with a DNA sequencer ABI-3100 in Example 3.
Figure 9 is a view showing the results of detection by ELISA method in Reference Example 1: where (a) is a picture showing the appearance of color development in each well and (b) is a graph showing the results of measurement with an ELISA reader.
Figure 10 is a chart showing the results of detection according to the detection method of the present invention in Reference Example 1 (results of GeneScan analysis using DNA sequencer ABI-3100).
Figure 11, similar to Figure 10, is a chart showing the results of detection according to the detection method of the present invention in Reference Example 1 (results of GeneScan analysis using DNA sequencer ABI-3100).
Figure 12 is a chart showing the results of detection according to the detection method of the present invention in Reference Example 2 (results of GeneScan analysis using DNA sequencer ABI-3100).
Figure 13 is a graph showing the plots of the detection results shown in Figure 12 as well as correlation of the amounts of protein (amount of antigen) with the detected values (signal values).

### DESCRIPTION OF THE REFERENCE NUMERALS

1: antigen, 2: well plate, 3: labeled antibody, 4: labeled antibody, 5: nucleotide chain, 6: nucleotide chain, 7: restriction enzyme site, 8: restriction enzyme site, 9: antigen-antibody complex, 10: antigen-antibody complex, 11: nucleotide fragment, 12: nucleotide fragment, 13: labeled antibody, 14: labeled antibody, 15: nucleotide chain, 16: nucleotide chain, 17:antigen-antibody complex, 18: antigen-antibody complex, 19: F primer, 20: R primer, 21: nucleotide fragment (PCR product), 22: nucleotide fragment (PCR product).

### BEST MODES FOR CARRYING OUT THE INVENTION

Hereinafter, the detection method of the present invention will be described in detail. The description, however, does not limit the scope of the present invention and the present invention can be appropriately modified and carried out apart from the following embodiments without departing from the spirit of the invention.

All of the prior art documents, patent publications, patent applications and other patent documents cited herein are incorporated herein by reference.

### 1. Summary of the present invention

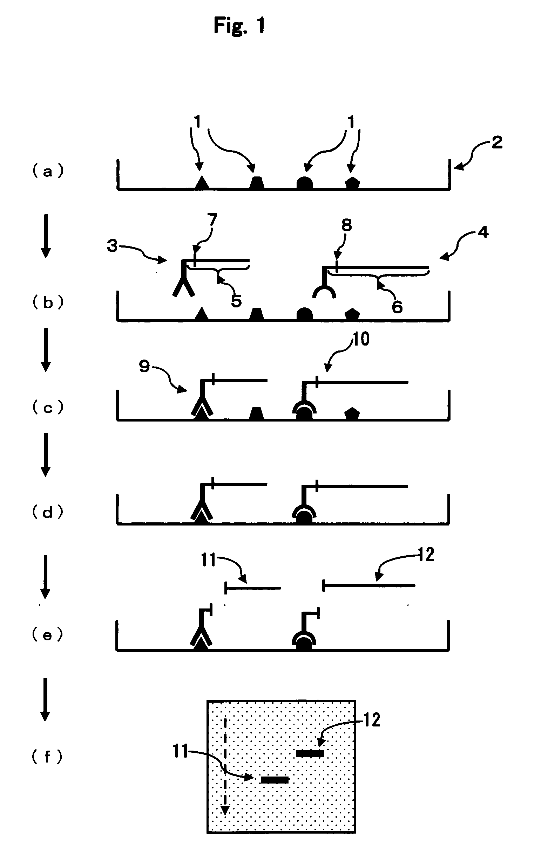

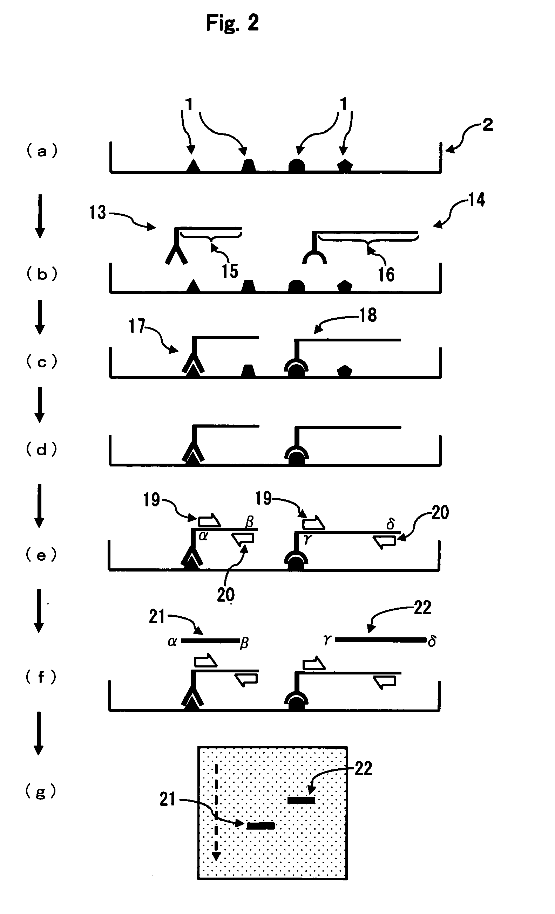

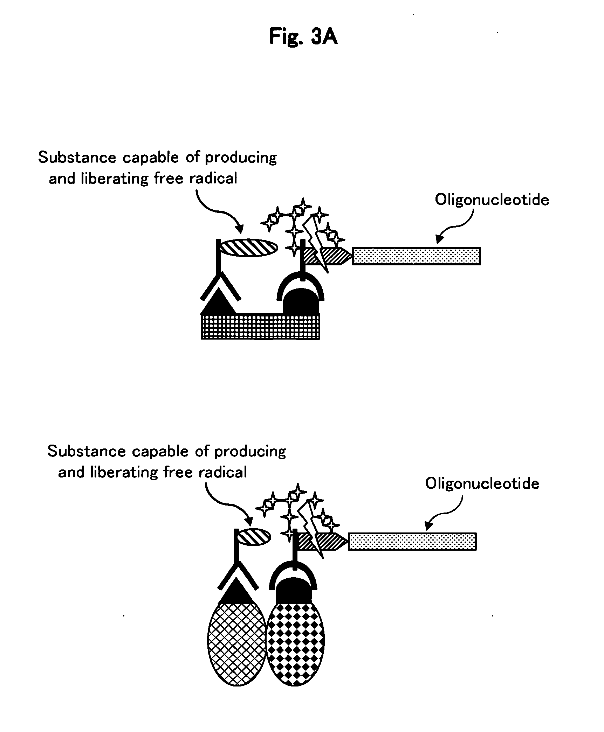

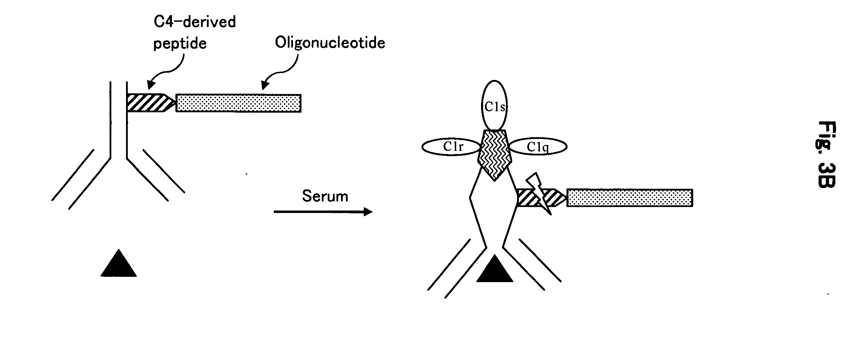

As described above, the detection method of the present invention comprises a step of forming a target substance-binder complex, and a step of detecting a label. First, the following four embodiments will be described for illustrating the general outline of the overall method of the present invention, and then each step will be described in detail. Although antigens and antibodies are used as exemplary target substances and binders in all of the following embodiments (1)-(4), the same can be applied when the target substance and the binder are other than antigens and antibodies.
(1) According to the first embodiment, as can be appreciated from the schematic flow diagram shown in Figure 1, first, multiple types (four types in Figure 1) of antigens 1 as target substances are immobilized on a well plate 2 as a support (Figure 1 (a)), and oligonucleotide-conjugated antibodies (labeled antibodies) 3 and 4 are added as binders to these antigens 1 (Figure 1(b)). The antibody 3 is forming a conjugate with an oligonucleotide chain 5 while the antibody 4 is forming a conjugate with an oligonucleotide chain 6. The antibodies 3 and 4 have common restriction enzyme sites 7 and 8 (e.g., EcoRI, etc.) in the oligonucleotide chains 5 and 6, respectively. The lengths of the oligonucleotide chains 5 and 6 (nucleotide sequences) are designed (or selected) such that the lengths of their terminal moieties from the restriction enzyme sites 7 and 8 are different. In Figure 1, the oligonucleotide chain of the antibody 3 is shorter.
   Next, the antibodies 3 and 4 are bound to specific antigens, respectively through antigen-antibody reaction to form antigen-antibody complexes 9 and 10 (Figure 1(c)). Antigens that did not form these complexes are washed away (Figure 1(d)).
   Subsequently, a restriction enzyme is added and reacted for cleaving the restriction enzyme sites 7 and 8, to obtain nucleotide fragments 11 and 12 of different lengths from the oligonucleotide chains 5 and 6 in the antibodies 3 and 4 forming the antigen-antibody complexes 9 and 10 (Figure 1(e)). The lengths of the obtained fragments are identified and detected by electrophoresis method using agarose gel or the like (Figure 1(f)).
   In Figure 1(f), two types of bands of different lengths corresponding to the nucleotide fragments 11 and 12 are detected. This confirms that two types of antigens to be detected are contained in the specimen containing four types of antigens.
(2) According to the second embodiment, as can be appreciated from the schematic flow diagram shown in Figure 2, first, multiple types (four types in Figure 2) of antigens 1 are immobilized as target substances on a well plate 2 as a support (Figure 2(a)), and oligonucleotide-conjugated antibodies (labeled antibodies) 13 and 14 are added as binders to these antigens 1 (Figure 2(b)). The antibody 13 is forming a conjugate with an oligonucleotide chain 15 while the antibody 14 is forming a conjugate with an oligonucleotide chain 16. The antibodies 13 and 14 each has a sequence to which common PCR primers (F primer 19 and R primers 20) can bind in the oligonucleotide chains 15 and 16, respectively. The lengths of the oligonucleotide chains (nucleotide sequences) 15 and 16 are designed (or selected) such that the lengths of the sequences (PCR sequences) amplified by PCR (Immuno-PCR) are different. Specifically, as shown in Figures 2(e) and 2(f), the sequence between alpha and beta is amplified in the oligonucleotide chain 15 of antibody 13 while the sequence between gamma and delta is amplified in the oligonucleotide chain 16 of antibody 14, where the sequence between alpha and beta is shorter.
   Next, the antibodies 13 and 14 are bound to specific antigens, respectively through antigen-antibody reaction to form antigen-antibody complexes 17 and 18 (Figure 2(c)). Antigens that did not form these complexes are washed away (Figure 2(d)).
   Subsequently, the PCR primers (F primer 19 and R primer 20) are used for PCR (Immuno-PCR) (Figure 2(e)) to obtain, as PCR products, nucleotide fragments 21 (fragment of alpha-beta amplified sequence) and 22 (fragment of gamma-delta amplified sequence) of different lengths from the oligonucleotide chains 15 and 16 in the antibodies 13 and 14 forming the antigen-antibody complexes 17 and 18, respectively (Figure 2(f)). The lengths of the obtained fragments are identified and detected by electrophoresis method using agarose gel or the like (Figure 2(g)).
   In Figure 2(g), two types of bands of different lengths corresponding to the nucleotide fragments 21 and 22 are detected. This confirms that two types of antigens to be detected are contained in the specimen containing four types of antigens.
(3) According to the third embodiment, as can be appreciated from the upper schematic view shown in Figure 3A, for a specific antigen, antibodies (a set of antibodies) that recognize different epitopes of the antigens are prepared. Specifically, one antibody is conjugated with an enzyme or a chemical substance capable of producing superoxide or other free radical with HRP, nitric oxide synthase, photosensitizer or the like. The other antibody is conjugated with an oligonucleotide chain or the like via a cross-linker (e.g., N-hydroxysuccinimide-4-azidobenzoate (HSAB)) that is cleavable with superoxide or other free radical. The oligonucleotide chain or the like in the other antibody is designed to have different length depending on the type of antigen to be recognized by the antibody.
   First, the prepared antibodies (binders) are added to multiple types of antigens (target substances) immobilized and/or nonimmobilized on a support (a well plate, etc.).
   Next, the antibodies are allowed to bind to specific antigens through antigen-antibody reaction to form antigen-antibody complexes. Antigens or labeled antibodies that did not form these complexes are washed away or left unwashed depending on the detection purpose.
   Next, a reagent that produces superoxide or other free radical is added. As a result, fragments containing oligonucleotide chains of different lengths corresponding to the respective antigens can be obtained. The lengths of the obtained fragments are identified and detected by electrophoresis method using agarose gel or with a microchip or the like.
   Furthermore, as shown in the schematic view (lower panel) in Figure 3A, this third embodiment can be employed to confirm the reaction of complex formation. Examples of such formation reactions include dimerization of a cell membrane receptor and intracellular protein binding. Thus, techniques that could to be necessary now or in the future for diagnosis of cancer or the like or for clarification of pathogenic mechanism can be provided.
   According to the third embodiment, the detection environment may be either a liquid phase or a solid phase.
(4) According to the fourth embodiment, as can be appreciated from the schematic view shown in Figure 3B, first, antibodies (labeled antibodies) as binders are added to multiple types of antigens (target substances) immobilized and/or nonimmobilized on a support (well plate, etc.). These antibodies are hybrid-chain-conjugated antibody conjugates in which an oligopeptide chain moiety and an oligonucleotide chain moiety are coupled to each other. The oligopeptide chain moiety is designed to have a site that can be cleaved with a certain protease while the oligonucleotide chain moiety is designed to have a different length according to the type of antigen to be recognized by the antibody. Next, the labeled antibodies are allowed to bind to specific antigens through antigen-antibody reaction to form antigen-antibody complexes. Antigens or labeled antibodies that did not form these complexes are washed away or left unwashed depending on the detection purpose.

Next, antibodies or complements (C1, etc.) that specifically recognize the formed antigen-antibody complexes and that are conjugated with the specific protease are added. When such an antibody or complement binds to an antigen-antibody complex, the protease cleaves the oligopeptide chain moiety in the near antigen-antibody complex. As a result, fragments containing the oligonucleotide chains of different lengths corresponding to different antigens are obtained. The lengths of the obtained fragments are identified and detected by electrophoresis method using agarose gel or with a microchip or the like.

Unlike a known sandwich technique, this fourth embodiment can be employed to detect an antigen of interest with an antibody that recognizes one type of epitope of the antigen.

According to the fourth embodiment, the detection environment may be either a liquid phase or a solid phase.

### 2. Step of forming target substance-binder complexes

The detection method of the present invention comprises a step of bringing multiple types of target substances immobilized and/or nonimmobilized on a support in contact with labeled binders capable of identifying according to the types of the target substances, thereby forming complexes between the target substances and the binders. The "binder" refers to a substance capable of specifically binding with a certain target substance, an example being an antibody corresponding to an antigen (a target substance).

### (1) Support

A support used with the present invention may be one on which a target substance such as an antigen can be immobilized and it is not limited as long as a binder (solution) such as an antibody can make contact to the target substance. Generally, as such a support, an insoluble material or structure is used. Preferably, examples include a support that can be used in an assay system of antigen-antibody reaction, which is specifically, a plastic multi-well plate, plastic beads, latex beads, magnetic beads, a plastic tube, a nylon membrane or a nitrocellulose membrane.

### (2) Target substance

The target substances intended to be detected by the method of the present invention are multiple types of target substances contained in a specimen (specifically, the same specimen). There is no limitation to the number of types of the target substances. Examples of such target substances include various proteins (including antibody proteins), peptides (oligopeptide, polypeptide, etc.), polysaccharides, glycolipids, various nucleic acids (DNAs and RNAs) and other low-molecular chemical synthetic materials and biological substances, among which those that can act as antigens (i.e., those to which antibody proteins are available or producible) are preferable. In another embodiment, an assay system may use multiple types of labeled binders for detecting one type of target substance to determine a binder specific to the target substance (i.e., to specify the type of the target substance).

Examples of the specimens include but not limited to biological substances (tissues and blood), foods such as meats and vegetables, soil, river water, fuel waste and the like.

Although the number of the types of target substances contained in the specimen is not limited as long as it is plural (at least 2), according to the method of the present invention, 10 or more types of specific target substances can clearly be identified and detected. Also, the number of the types of target substances may be 50 or more or 100 or more.

The concentration of the target substances in the specimen is not limited. For example, according to the present invention, a specific target substance can clearly be identified and detected when the concentration of the target substance per 1 µL of the specimen is in the order of ng or lower. The concentration may also be in the order of pg or lower or in the order of fg or lower. Specifically, when the following binders (i)-(iv) (described below) are used as the labeled binders, they can be identified and detected with high sensitivity even when the concentrations of the target substances are lower. Among which, the binders of (ii) or (iii) are preferably used.
(i) A binder forming a conjugate with an oligonucleotide chain having a region amplifiable by PCR.
(ii) A binder forming a conjugate with an oligonucleotide chain or an oligopeptide chain bound to a fluorescent dye.
(iii) A binder forming a conjugate with an oligonucleotide chain or an oligopeptide chain incorporating a radioisotope element or bound to a radioactive substance.
(iv) A binder forming a conjugate with an oligonucleotide chain or an oligopeptide chain bound to a labeled dendrimer.

According to the method of the present invention, but without limitation, a target substance is immobilized on a support beforehand and brought into contact with a labeled binder (solution), by which specific binding reaction may take place between the target substance and the binder, or the reaction may take place without immobilizing the target substance on a support, or the reaction may take place by a combination thereof.

Examples of methods for immobilizing a target substance on a support include but not limited to a method in which the target substance is directly immobilized on the surface of a support, and a method in which an antibody that specifically binds to the target substance is bound to the surface of a support and then the target substance is bound to the immobilized antibody for indirect immobilization. According to the latter indirect immobilization method, since substances to be detected may be selected beforehand among the various substances as possible target substances, detection sensitivity and detection accuracy can be enhanced. In the latter immobilization method, if the labeled binder used later is also an antibody, the antibody directly immobilized on the support usually has an epitope that recognizes a different target substance (antigen) from that of the antibody used later.

Preferably, when a target substance is to be immobilized on a support, the target substance is subjected to blocking according to a conventional method before bringing it into contact with the labeled binder (solution). In the case of direct immobilization, blocking is preferably performed after the immobilization while in the case of indirect immobilization, blocking is preferably performed after immobilizing the antibody to the support and before binding to the target substance.

### (3) Binding substance

According to the method of the invention, multiple types of labeled binders are used to identify the respective types of target substances. One kind of labeling treatment is performed for each specific binder corresponding to a particular target substance such that the numbers and the types of the labels can be specified in the later detection stage, thereby specifying the numbers and the types of the detected target substances. The present invention is not limited to this and, for example, the same labeling treatment may be performed on multiple types of binders to collectively detect multiple types of target substances.

Other than an antibody (antibody protein) that can specifically bind to a particular antigen substance, the binder that may be used in the method of the present invention may be, for example, a single-stranded nucleic acid (DNA, RNA (mRNA, etc.), synthetic nucleic acid) that can hybridize to a particular target gene or a nucleic acid molecule, various proteins (except an antibody) that can specifically bind to a particular target protein or the like, a protein (lectin, etc.) that can specifically bind to a particular glycolipid, and an antigen substance that can specifically bind to a particular antibody, among which an antibody is preferable.

Generally, when the binders are antibodies, monoclonal antibodies having specificity to respective types of antigens (target substances) are preferably used although the present invention is not limited thereto. For example, a monoclonal antibody having a specificity common to particular multiple types of antigens may also be used. In an assay system using such an antibody, multiple types of antigens may collectively be detected with a single antibody.

According to the method of the present invention, a phrase "bringing a target substance immobilized on a support in contact with a labeled binder" generally refers to binding via a direct contact between the target substance and the labeled binder. The present invention, however, is not limited thereto, and in a broader sense, it also includes, first binding a primary binder (primary antibody, etc.) having a specificity to a target substance to the target substance immobilized on a support and then binding the labeled binder described above as a binder having a specificity to this primary binder so that eventually the target substance is indirectly bound to the labeled binder. In this indirect binding, the primary binder may further be bound to a secondary binder, a tertiary binder, ..., an n-th binder, in which case the labeled binder is one that specifically binds to the n-th binder. Here, "n" is 1 to 11, preferably 1 or 2.

Preferably, the binder used in the method of the present invention is one forming a conjugate with any type of nucleic acid chain, peptide chain or the like as a label substance. When the binder is an antibody, the conjugate is referred to as a "conjugated antibody". Preferable examples of the nucleic acid chain, the peptide chain or the like include oligonucleotide chains (DNA chain, RNA chain), oligopeptide chains and oligopeptide nucleic acid chains, which may be either natural or synthetic. In particular, a binder forming a conjugate with an oligonucleotide chain (oligonucleotide conjugate) is more preferable since it allows identifications that are highly useful and highly practical such as identification by the lengths of nucleotide sequences or oligonucleotide chains, identification by fluorescent dye binding, incorporation of radioisotope elements or the like, or identification by the lengths of fragments amplified by PCR. A binder forming a conjugate with a synthesized oligonucleotide chain is more preferable.

The lengths of the nucleic acid chains, peptide chains or the like are not particularly limited.

For example, in the case of oligonucleotide chains (DNA chains, RNA chains), the length is preferably 100-5,000 mers, more preferably 100-1,000 mers, and still more preferably 100-500 mers. If the length of the oligonucleotide chain is within the above range, formation of a conjugate with a binder becomes easy, and thus effective in stabilizing the state after the formation of the conjugate, in enhancing the detection sensitivity, in reducing the time for detection and the like.

For example, in the case of an oligopeptide chain, the length is preferably 10-1,000 amino acid residues, more preferably 10-500 amino acid residues, and still more preferably 10-100 amino acid residues. If the length of the oligopeptide chain is within the above range, formation of a conjugate with a binder becomes easy, and thus effective in stabilizing the state after the formation of the conjugate, in facilitating the labeling treatment of the oligopeptide chain and the like.

For example, in the case of an oligopeptide nucleic acid chain, an oligopeptide chain having 10-100 amino acid residues is preferably bound to a nucleic acid chain (DNA chain, RNA chain) having a length of 100-5,000 mers, more preferably the nucleic acid chain moiety of 100-1,000 mers, and still more preferably the nucleic acid chain moiety of 100-500 mers. If the length of the oligopeptide nucleic acid chain is within the above range, formation of a conjugate with a binder becomes easy, and thus effective in stabilizing the state after the formation of the conjugate, in enhancing the detection sensitivity, in reducing the time for detection and the like.

The oligonucleotide conjugate above can be obtained, for example, by covalently binding one end of an oligonucleotide chain (DNA chain, RNA chain) as a label moiety to a binder. Similarly, the oligopeptide conjugate and the oligopeptide nucleic acid conjugate can be obtained, for example, by covalently binding one end of an oligopeptide chain or an oligopeptide nucleic acid chain as a label moiety to a binder. In preparing these conjugates, an oligonucleotide chain, an oligopeptide chain or an oligopeptide nucleic acid chain may be introduced with, for example, one, two or more thiol or amino groups (substituents) or biotin (or avidin) or the like by chemical or enzymatic treatment (preferably chemical treatment). This is effective in facilitating formation of a conjugate with a binder, in further stabilizing the state after the formation of the conjugate, in enhancing the yield of the obtained conjugate and thus in enhancing the detection sensitivity or the detection effects.

Examples of the methods for preparing the oligonucleotide conjugate above include: (i) a method comprising binding an oligonucleotide chain introduced with an amino group or a thiol group at the 5' terminal end to a binder using a bifunctional cross-linker (E. Hendrickson et al., Nucl. Acids Res., Vol 23 (3), p522-529 (1995)); and (ii) a method comprising biotinylating both of the binder and the oligonucleotide chain beforehand, mixing the binder with the oligonucleotide chain and adding avidin thereto to bind the oligonucleotide chain to the binder via avidin.

Examples of the methods for preparing the oligopeptide conjugate and the oligopeptide nucleic acid conjugate include: a method comprising binding (i) an oligopeptide chain or an oligopeptide nucleic acid chain (hereinafter, oligopeptide chain or the like) having an amino group or a thiol group; (ii) an oligopeptide chain or the like introduced with an amino group or a thiol group, to a binder using a bifunctional cross-linker; and (iii) a method comprising biotinylating both of the binder and the oligopeptide chain or the like beforehand, mixing the binder with the oligopeptide chain or the like and adding avidin thereto to bind the oligopeptide chain or the like to a binder via avidin.

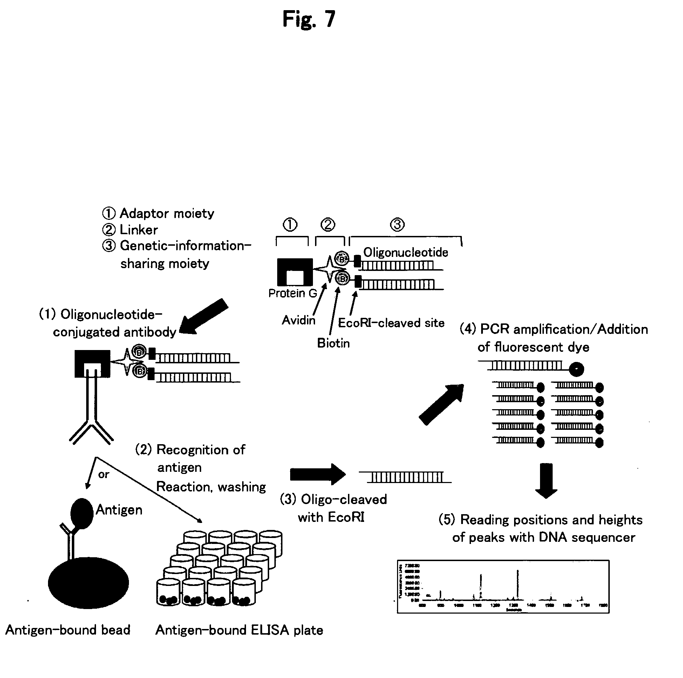

Moreover, according to the present invention, the various types of conjugates described above may be obtained through formation of conjugates between an oligonucleotide chain, an oligopeptide chain or an oligopeptide nucleic acid as a label moiety and a binder via at least an adapter moiety (see Figure 7(1)). Binding via an adapter moiety can be effective in further enhancing the structural stability of the conjugate, in enhancing the yield of the resulting conjugate, and consequently in enhancing detection sensitivity and detection effects. Examples of the adapter moieties include various proteins such as protein G, protein A, protein L and a fusion protein of protein A and protein G, which are preferable especially when the binder is an antibody molecule since they can bind easily and stably to the antibody.

A method for preparing a conjugate including an adapter moiety is preferably but not limited to a method comprising (i) first, binding an oligonucleotide chain or the like as a label moiety to an adapter moiety; and (ii) then binding the adapter moiety to a binder.

Specifically, in (i) above, the adapter moiety is modified with avidin, the oligonucleotide chain or the like as the label moiety is biotinylated, and both are mixed together to bind the oligopeptide chain or the like to the adapter moiety. Alternatively, both of the adapter moiety and the oligopeptide chain or the like are biotinylated beforehand, then the adapter moiety and the oligopeptide chain or the like are mixed together and added with avidin to bind the oligopeptide chain or the like to the adapter moiety via avidin. In the former approach, avidin modification of the adapter moiety may comprise first reacting and binding a linker compound with an adapter moiety and then binding the compound with avidin. When the adapter moiety used is protein A, G or L, "sulfosuccinimidyl 4-(N-maleimidomethyl) cyclohexane-1-carboxylate (Sulfo-SMCC)" or the like may preferably be used as the linker compound.

Next, in (ii) above, when the adapter moiety has binding reactivity with the binder, the adapter moiety/label moiety binding unit obtained in (i) above can be mixed with the binder to obtain various conjugates such as oligonucleotide conjugates. When the adapter moiety has no binding reactivity with the binder in the first place, both can be biotinylated and then mixed together in the presence of avidin to obtain various conjugates using similar techniques that may be employed in (i) above.

In (3-1) and (3-2) below, labeling treatments for an oligonucleotide conjugate or the like and an oligopeptide conjugate will specifically be illustrated. Although all of these illustrations use antigen and antibody as exemplary target substances and binders, the same description applies to the case where the target substance and the binder are other than an antigen and an antibody.

### (3-1) Labeling treatment for oligonucleotide-conjugated antibody

In the case of an oligonucleotide-conjugated antibody, the labeling treatment may be performed on but not limited to an oligonucleotide chain itself (e.g., the treatments of (A1)-(A3), (A6), (A7) and (A9) below, etc.) or via an oligonucleotide chain (e.g., the treatments of (A4), (A5) and (A8) below, etc.).

Specifically, the labeling treatment performed on the oligonucleotide chain is preferably at least one treatment selected from the following (A1)-(A9). In particular, treatments of (A1), (A2) and (A9) are preferable in that the labeling treatment is easy and that detection sensitivity is high.
(A1) Treatment of providing a site cleavable with a restriction enzyme (see Figure 1).
(A2) Treatment of providing a region amplifiable by PCR (see Figure 2).
(A3) Treatment of incorporating a radioisotope element.
(A4) Treatment of binding a fluorescent dye.
(A5) Treatment of binding an enzyme.
(A6) Treatment of providing a site cleavable by light irradiation.
(A7) Treatment of providing a site cleavable with active oxygen (see Figure 3A).
(A8) Treatment of binding a labeled dendrimer.
(A9) Treatment of providing a difference by at least one base type.

Moreover, in the case of an oligopeptide nucleic acid-conjugated antibody, a labeling treatment similar to that for the oligonucleotide-conjugated antibody described above may be performed on the nucleic acid (oligonucleotide) moiety thereof. The following specific descriptions similarly apply to each of the actual labeling treatments.

In the case of the treatment of (A1) above, for example, as shown in Figure 1, a label can be identified and detected by making the length of the cleaved oligonucleotide fragments thereof to be different from those of the labels of other antibodies, by making no oligonucleotide fragment to be cleaved from the labels of other antibodies, or by a combination thereof.

Specifically, the lengths of the cleaved fragments can be made different through synthesis such that the lengths of the oligonucleotide chains bound to the antibodies are substantially the same among the antibodies but the positions of sites cleaved by a restriction enzyme are different from each other, or such that the lengths of the oligonucleotide chains themselves bound to the antibodies are made different from each other. The difference of the lengths of the cleaved fragments is not particularly limited but is preferably 10 mers or longer, more preferably 50 mers or longer, and still more preferably 100 mers or longer for identification and detection with good sensitivity.

In the case of the treatment of (A2) above, for example, as shown in Figure 2, a label can be identified and detected by making the length of the fragment obtained as the PCR product different from that of the labels of other antibodies, by making no PCR product to be generated from the labels of other antibody, or by a combination thereof.

Specifically, the lengths of the fragments obtained as PCR products may be made different through synthesis such that the binding sites of the primer are different although the same primer is used and the widths (lengths) of the regions amplifiable by PCR are different among the antibodies. The difference of the lengths of the fragments of the PCR products is not limited but is preferably 5 mers or longer, more preferably 10 mers or longer, and still more preferably 50 mers or longer for identification and detection with good sensitivity.

In the case of the treatment of (A3) above, for example, the label may be identified and detected by making the radiation type of the label different from those of the labels of other antibodies, by making the labels of other antibodies unable to produce radiation, or by a combination thereof.

The method of the treatment of incorporating a radioisotope element may be, for example, a method that uses a nucleic acid that incorporates a radioisotope element upon synthesizing an oligonucleotide chain, or a method in which an oligonucleotide chain is directly labeled with a modifying group (³²PO₄, ³⁵SO₄) or H¹²⁵I incorporating a radioisotope element or the like.

Specifically, radioisotope elements may be ³²P, ³H, ¹⁴C, ³⁵S, ⁵⁹F, ¹²⁵I or the like, among which, in order to enhance the sensitivity of identification and detection, a combination of a radioisotope element that emits beta ray (e.g., ³H or ¹⁴C) and a radioisotope element that emits gamma ray (e.g., ¹²⁵I) is preferably used.

In the case of the treatment of (A4) above, for example, the label may be identified and detected by making the maximum absorption wavelength of the fluorescent dye for that label different from those for the labels of other antibodies, by making the fluorescent dye unable to bind to the labels of other antibodies, or by a combination thereof.

A method of the treatment of binding a fluorescent dye may be, for example, a method in which a functional group of an oligonucleotide chain is directly labeled with a fluorescent dye.

Specifically, as the fluorescent dye, fluorescein isothiocyanate (FITC), tetramethylrhodamine isothiocyanate (RITC), luciferase, GFP (including EGFP with different fluorescence intensity, etc.), derivatives thereof (YFP, BFP or the like with different fluorescence wavelengths and different excitation wavelengths) or the like may be used. In particular, in terms of enhancing the sensitivity of identification and detection, a combination of FITC and RITC or a combination of GFP and a derivative thereof are preferably used.

In the case of the treatment of (A5), the label may be identified and detected, for example, by using an enzyme that produces a fluorescent dye by binding with a color developing substrate, which is different from enzymes for the labels of other antibodies so that the maximum absorption wavelengths of the fluorescent dyes are different, by making the enzyme unable to bind to the labels of other antibodies, or by a combination thereof.

A method of the treatment of binding an enzyme may be, for example, a method in which a salicylhydroxyaminated enzyme is bound to an oligonucleotide chain via a protein cross-linker.

Specifically, as the enzyme, alkaline phosphatase, various peroxidases (horseradish peroxidase), beta galactosidase, xanthine oxidase or the like may be used. In particular, in terms of enhancing the sensitivity of identification and detection, a combination of DAB and TMB is preferably used as sensitizers in the color development technique.

In the case of the treatment of (A6) above, the label may be identified and detected, for example, by making the cleavable light wavelength different from those for the labels of other antibodies, by giving different identification treatments such as making the lengths of the cleaved fragments different while the cleavable light wavelength is the same for those of the labels of other antibodies, by making the labels of other antibodies unaffected by cleavage by light irradiation, or by a combination thereof.

Specifically, a cross-linker (bifunctional cross-linker, etc.) cleavable by irradiation with light having a predetermined wavelength is added to an oligonucleotide chain, and then the oligonucleotide chain is bound to an antibody via this linker. The cross-linker cleavable by light irradiation may be, for example, an oxime ester derivative, a carbamoyloximino derivative or the like. When the cleavable light wavelength is made different from those for the labels of other antibodies, crosslinking may be carried out by using a cross-linker having a different wavelength from those of other antibodies. In this case, the cross-linker is preferably a combination of an oxime ester derivative and a carbamoyloximino derivative in terms of good detection sensitivity and the like. When the length of the cleaved fragment is made different from those of the labels of other antibodies, the lengths of the oligonucleotide chains bound via a cross-linker are made different. The difference of the lengths of the cleaved fragments is not limited but preferably 10 mers or longer, more preferably 50 mers or longer, still more preferably 100 mers or longer in terms of identification and detection with good sensitivity. Moreover, different labeling treatment may be carried out on the cleaved fragment of the label from those for the labels of other antibodies by performing treatments (A3)-(A5) above and (A8) below on the oligonucleotide chains bound via the cross-linker.

In the case of the treatment of (A7) above, the label may be identified and detected, for example, by performing different labeling treatments such that length of the cleaved fragment is different from those of the labels of other antibodies, by making the labels of other antibodies unaffected by cleavage by active oxygen, or by a combination thereof.

Specifically, a cross-linker (bifunctional cross-linker, etc.) cleavable by bringing it into contact with active oxygen is added to an oligonucleotide chain, and then the oligonucleotide chain is bound to an antibody via this linker. The cross-linker cleavable by contact with active oxygen may be, for example, an azide cross-linker such as N-hydroxysucciniimide-4-azidobenzoate, an S-nitrosamine derivative, an N-nitrosamine derivative or the like.

The length of the cleaved fragment can be made different from those of the labels of other antibodies, for example, by making the length of the oligonucleotide chain bound via the cross-linker different. The difference of the lengths of the cleaved fragments is not limited but preferably 10 mers or longer, more preferably 50 mers or longer, still more preferably 100 mers or longer in terms of identification and detection with good sensitivity. Furthermore, a labeling treatment different from those for the labels of other antibodies may be performed on the cleaved fragment, for example, by performing the treatments of (A3)-(A5) above and (A8) below on the oligonucleotide chain bound via a cross-linker.

In the case of the treatment of (A8) above, for example, dendrimers subjected to 1 or 2 types of labeling treatments can be used, which can be obtained by subjecting various dendrimers such as oligonucleotide dendrimers (DNA dendrimer, RNA dendrimer) to treatments such as incorporating a radioisotope element, binding a radioactive substance, binding a fluorescent dye or binding an enzyme as described above. The label can be identified and detected by making the type, degree or the like thereof different from those of the labels of other antibodies, by making the labels of other antibodies unable to bind to such a labeled dendrimer, or by a combination thereof.

A dendrimer refers to a dendritic multibranched polymer comprised of components thereof (e.g., oligonucleotides, DNAs, etc.) in which regularly branched backbone structures are spreading three-dimensionally from the center (core). A dendrimer has repeat units with a predetermined chemical bond between the branch points. In general, the number of the repeats is expressed in "generation". The larger the number of generation, the larger and the more sphere-like the structure becomes.

According to the present invention, similar to the labeled dendrimer, a labeled dendron may also be used. A dendron refers to a dendritic multibranched polymer similar to a dendrimer but which spreads (extends) only in one direction from the center (focal point).

Examples of methods for synthesizing a dendrimer include but not limited to a divergent approach that develops synthesis from the core to the periphery, a convergent approach that develops synthesis from a terminal functional group to the core and a combination thereof. These methods similarly apply to the case of dendron. Since the synthetic pathway of a dendrimer or a dendron is well-ordered, a labeling substance or the like as described above may be introduced in a three-dimensional manner to a desired position either inside or outside the dendrimer or dendron.

Examples of methods of a treatment of binding a labeled dendrimer include a method in which a sequence region (arm) that can complementary bind to an oligonucleotide chain binding to an antibody is provided at the terminal (the outermost layer) of the dendrimer, to which the oligonucleotide chain is bound by hybridization under predetermined conditions, and a method in which an amino group or a thiol group is added to an unbound terminal of an oligonucleotide chain binding to an antibody, to which the terminal of the dendrimer is bound using a bifunctional cross-linker. The same applies to a method of a treatment of binding a labeled dendron.

In the case of the treatment of (A9) above, first, each of the oligonucleotide chains attached to respective conjugated antibodies is provided with at least one site having a different base type while they may have similar lengths (lengths whose difference cannot be recognized by the treatments (A1) and (A2) above). Then, these PCR amplified fragments containing different types of bases can be identified and detected by an analysis that can recognize the difference by one base type such as an analysis with a DNA sequencer (e.g., product name: ABI-3100, Applied Biosystems) or an analysis by real time PCR using ABI PRISM 7000 Sequence Detection System (Applied Biosystems) or the like. In order to liberate a predetermined fragment as a template before PCR amplification, each oligonucleotide chain may be provided with a restriction enzyme site (preferably, a cleavage site made by the same restriction enzyme). In this treatment, the variations of the labels are possible in a number of combinations calculated as "[types of bases (A, T, G, C, etc.)] x [lengths of bases (number of bases)]". This treatment is particularly preferable for simultaneous multiple detections. The variations above can readily be designed by using various types of known mutagenesis and by providing a difference to the nucleotide sequences at the stage of synthesizing nucleic acids.

### (3-2) Treatment of labeling oligopeptide-conjugated antibody

In the case of the oligopeptide-conjugated antibody, the labeling treatment may be performed on but not limited to the oligopeptide chain itself (e.g., treatments of (B1), (B2), (B5) and (B6) below) or via the oligopeptide chain (e.g., treatments of (B3), (B4) and (B7) below).

Specifically, the labeling treatment above performed on the oligopeptide chain preferably include at least one treatment selected from (B1)-(B7) below, among which the treatment of (B1) is more preferable in terms of easy labeling treatment, high detection sensitivity or the like.
(B1) Treatment of providing a site cleavable with protease.
(B2) Treatment of incorporating a radioisotope element.
(B3) Treatment of binding a fluorescent dye.
(B4) Treatment of binding an enzyme.
(B5) Treatment of providing a site cleavable by light irradiation.
(B6) Treatment of providing a site cleavable with active oxygen (see Figure 3A).
(B7) Treatment of binding a labeled dendrimer.

In the case of the oligopeptide nucleic acid-conjugated antibody, the same labeling treatment as that in the case of the oligopeptide-conjugated antibody above may be performed on the oligopeptide moiety thereof. The specific description below may appropriately be applied to each of the actual labeling treatments.

In the case of the treatment of (B1) above, for example, the label may be identified and detected by making the lengths of the oligopeptide fragments obtained after the cleavage different from those of the labels of other antibodies by making the labels of other antibodies unable to generate the oligopeptide fragment after the cleavage, or by a combination thereof.

Specifically, in order to make the lengths of the cleaved fragments different, the lengths of the oligopeptide chains to be bound to the antibodies are made substantially the same among the antibodies but are synthesized such that their positions of cleavage sites by restriction enzymes are different, or the lengths of the oligonucleotide chains to be bound to the antibodies are made different among the antibodies. The difference of the lengths of the fragments after the cleavage is not particularly limited but is preferably 5 amino acid residues or more, more preferably 10 amino acid residues or more, still more preferably 20 amino acid residues or more for identification and detection with good sensitivity.

In the case of the treatment of (B2) above, for example, identification and detection may be performed in the same manner as (A3) above.

Examples of methods of a treatment of incorporating a radioisotope element include a method using nucleic acids that incorporate the radioisotope element upon synthesizing the oligopeptide chain, and a method comprising directly labeling the oligopeptide chain with a modifying group (³²PO₄, ³⁵SO₄) or H¹²⁵I incorporating the radioisotope element.

Preferably, specific examples of the radioisotope elements and preferable combinations thereof are also the same as (A3) above.

In the case of the treatment of (B3) above, identification and detection may be performed, for example, as described in (A4) above.

An example of a method of a treatment of binding a fluorescent dye includes a method comprising directly labeling the functional group of the oligopeptide chain with the fluorescent dye.

Preferably, specific examples of the fluorescent dyes and combinations thereof are also the same as (A4) above.

In the case of the treatment of (B4) above, identification and detection may be performed, for example, as described in (A5) above.

An example of a method of a treatment of binding an enzyme includes a method comprising binding a salicylhydroxyaminated enzyme to the oligopeptide chain via a protein cross-linker.

Preferably, specific examples of the enzymes and preferable combinations of sensitizers are also the same as (A5) above.

In the case of the treatment of (B5) above, identification and detection can be performed, for example, in the same manner as (A6) above.

Specifically, a cross-linker (bifunctional cross-linker, etc.) cleavable by irradiation with light with a predetermined wavelength is added to the oligopeptide chain, and then the oligopeptide chain is bound to an antibody via this linker. The cross-linker cleavable by light irradiation may be, for example, the same as those of (A6) above. The cleavable light wavelength can be made different from those for the labels of other antibodies in the same manner as (A6) above including the combination of the cross-linkers. Also, the length of the fragment after cleavage can be made different from those of the labels of other antibodies in the same manner as (A6) above. The difference of the lengths of the fragments after cleavage is not limited and can be determined appropriately. Moreover, a different labeling treatment may be performed on the fragment after cleavage from labeling treatments on those of the labels of other antibodies by performing treatments (B2)-(B4) above and (B7) below on the oligopeptide chain which is bound via the cross-linker.

In the case of the treatment of (B6) above, identification and detection can be performed, for example, in the same manner as (A7) above.

Specifically, a cross-linker (bifunctional cross-linker, etc.) cleavable by contact with active oxygen is added to the oligopeptide chain, and then the oligopeptide chain is bound to an antibody via this linker. The cross-linker cleavable by contact with the active oxygen may be, for example, the same as those of (A7) above. The length of the fragment after cleavage can be made different from those of the labels of other antibodies in the same manner as (A7) above. The difference of the lengths of the fragments after cleavage is not limited and can be determined appropriately. Moreover, a different labeling treatment may be performed on the fragment after cleavage from those on the labels of other antibodies by performing treatments (B2)-(B4) above and (B7) below on the oligopeptide chain which is bound via the cross-linker.

In the case of the treatment of (B7) above, identification and detection can be performed, for example, in the same manner as (A8) above. In addition, a labeled dendron can also be used like the labeled dendrimer.

Examples of methods of a treatment of binding a labeled dendrimer include a method in which a sequence region (arm) that may complementary bind to an oligopeptide chain binding to an antibody is provided at the terminal (the outermost layer) of the dendrimer, which is then bound to the oligopeptide chain by hybridization under predetermined conditions, and a method in which an amino group or a thiol group is added to an unbound terminal of the oligopeptide chain binding to an antibody, which is then bound to the terminal of the dendrimer using a bifunctional cross-linker. The same applies to a method of a treatment of binding a labeled dendron.

### (4) Antigen-antibody reaction

As described above, labeled binders (solution) is generally brought into contact with target substances immobilized (coated) on a support by performing blocking treatment with a known blocking solution, and then washing thoroughly with a known washing solution such as PBS. Subsequently, a solution containing multiple types of labeled binders is added at a suitable amount for binding reaction between the target substances and the binders while agitating for 30-60 minutes at room temperature to allow formation of complexes of both substances. Preferably, the resultant is washed thoroughly again.

Preferably, when a labeled binder (solution) is brought into contact with a target substance that is not immobilized on a support or the like, generally, a specimen containing the target substance is appropriately pretreated to remove or reduce impurities other than the target substance.

### 3. Step of detecting labels

The detection method of the present invention comprises a step of detecting the labels in the binders forming the target substance-binder complexes. Specifically, since the detection methods differ according to the types of the labeling treatments of the binders as described above (labeling treatments (A1)-(A9) and (B1)-(B7) above), they will be described hereinafter according to their types.

### (1) In the case of treatment of providing site to be cleaved with restriction enzyme (treatment of (A1))

When an oligonucleotide conjugate (DNA conjugate or RNA conjugate) is used, a predetermined restriction enzyme solution is added to cleave the oligonucleotide chain according to a conventional method. The length of the obtained fragment can be identified and detected by electrophoresis method using agarose gel or the like. In addition, as shown in Figures 7(3)-(5), the free fragment treated by the restriction enzyme above is amplified by PCR using predetermined fluorescently-labeled primers, and the obtained amplified fragment is subjected to analysis by GeneScan software using a DNA sequencer (e.g., Applied Biosystems, product name: ABI-3100) for specifying the positions and heights of the peaks to identify and detect the length of the free fragment after the restriction enzyme treatment.

If necessary, when the antigen amount is low or the concentration of the fragment after the restriction enzyme treatment is low in the detection above, the treated reaction solution is preferably concentrated by centrifugal sedimentation using a spin column or the like.

When the oligopeptide nucleic acid conjugate is used, the same detection method as above may be employed for the nucleic acid chain (oligonucleotide chain) moiety thereof.

### (2) In the case of treatment of providing region amplifiable by PCR (treatment of (A2))

When an oligonucleotide conjugate (DNA conjugate or RNA conjugate) is used, according to a conventional method, predetermined primers designed are added to obtain a PCR product having a particular amplified fragment by PCR (about 5-30 cycles). The length of the obtained fragment can be identified and detected by electrophoresis method using agarose gel or the like. In addition, using fluorescently-labeled primers as the predetermined primers, the obtained amplified fragment can be subjected to an analysis by GeneScan software using a DNA sequencer (e.g., Applied Biosystems, product name: ABI-3100) for specifying the positions and heights of the peaks to identify and detect the length of the amplified fragment.

When the oligopeptide nucleic acid conjugate is used, the same detection method as above may be employed for the nucleic acid chain (oligonucleotide chain) moiety thereof.

### (3) In the case of treatment of providing site to be cleaved with protease (treatment of (B1))

When an oligopeptide conjugate is used, according to a conventional method, a predetermined protease solution is added to cleave the oligopeptide chain, and then the length of the fragment is identified and detected by an electrophoresis method such as SDS-PAGE. If necessary, when the amount of the target substance is low or the concentration of the fragment after the restriction enzyme treatment is low, the treated reaction solution is preferably concentrated by centrifugal sedimentation with a spin column or the like.

When the oligopeptide nucleic acid conjugate is used, the same detection method as described above may be employed for the nucleic acid chain (oligonucleotide chain) moiety thereof.

### (4) In the case of treatment of binding fluorescent dye (treatments of (A3) and (B2))

The predetermined fluorescence wavelength is identified and detected with a known fluorometer, fluorescence microscope or the like.

According to the present invention, a measurement module device equipped with a semiconductor laser, a high-sensitive photodiode or the like (e.g., Hitachi, Ltd., product name: cosmo-i; Applied Biosystems, product name: ABI-3100) may also be used. These devices are capable of separating/identifying and quantifying a small amount of DNA, RNA, peptide or the like in a short period of time. Use of this device allows identification and detection under simultaneous multiple detection conditions in a rapid (in a short period of time) and highly sensitive manner that has not been realized so far. Specifically, measurement time may be within 120 minutes (preferably within 60 minutes, more preferably within 30 minutes), the detection sensitivity may be 100-100,000 fg/mL (preferably 100-10,000 fg/mL, more preferably 100-1,000 fg/mL), and the detectable number of labels (per chip) may be 5 types or more (preferably 10 types or more, more preferably 50 types or more).

### (5) In the case of treatment of binding enzyme (treatments of (A4) and (B3))

According to a known ELISA method, a color developing substrate is added to produce a fluorescent dye, and then identification and detection can be carried out in the same method as the treatment of binding the fluorescent dye.

### (6) In the case of treatment of incorporating radioisotope element (treatments of (A5) and (B4))

Using a known radiation measuring instrument or according to a known conventional western blotting, a predetermined radiation type can be identified and detected.

### (7) In the case of treatment of providing cleavage site with light irradiation (treatments of (A6) and (B5))

Light with a predetermined wavelength is radiated to cleave an oligonucleotide chain, an oligopeptide chain or the like (more specifically, separate them from the binder). Then, the length of the obtained fragment is identified and detected by, for example, an electrophoresis method such as an electrophoresis method or SDS-PAGE using agarose gel or the like. If necessary, when the amount of the target substance is low or the concentration of the fragment after the light irradiation is low, the treated reaction solution is preferably concentrated by centrifugal sedimentation with a spin column or the like. Furthermore, the fragment after cleavage can be identified and detected in the same manner as (4)-(6) above.

### (8) In the case of treatment of providing site to be cleaved with active oxygen (treatments of (A7) and (B6))

First, a reagent that generates and liberates free radical such as HRP (horseradish peroxidase) and Fe complex is added to generate active oxygen. This active oxygen cleaves an oligonucleotide chain, an oligopeptide chain or the like (more specifically, separate them from the binder). Then, the length of the obtained fragment is identified and detected by, for example, an electrophoresis method such as an electrophoresis method using agarose gel or the like or SDS-PAGE. If necessary, when the amount of the target substance is low or the concentration of the fragment after the cleavage treatment is low, the treated reaction solution is preferably concentrated by centrifugal sedimentation with a spin column or the like. Furthermore, after this cleavage, identification and detection can be carried out in the same manner as (4)-(6) above.

### (9) In the case of treatment of binding labeled dendrimer (treatments (A8) and (B7))

Specifically, although the detection method differs according to the type of the labeling treatment performed on the dendrimer, identification and detection can be carried out in the same method as each of the labeling treatments described above. The same applies when a labeled dendron is used.

### (10) In the case of treatment of providing a difference by at least 1 base type (treatment of (A9))

When an oligonucleotide conjugate (DNA conjugate or RNA conjugate) is used according to a conventional method, a predetermined restriction enzyme solution is first added for cleaving the oligonucleotide chain. Alternatively, a PCR product having a particular fragment amplified is obtained by PCR (about 5-30 cycles) using a free fragment obtained by the restriction enzyme treatment as a template with predetermined primers. Alternatively, a PCR product is obtained in the same manner as described above without performing the restriction enzyme treatment described above by PCR directly using the oligonucleotide chain in the oligonucleotide conjugate as a template together with predetermined primers. Subsequently, identification and detection can be carried out based on the difference in the nucleotide sequences of the amplified fragments after PCR by an analysis capable of distinguishing by a single base such as an analysis with a DNA sequencer described above and an analysis by real time PCR.

When the oligopeptide nucleic acid conjugate is used, the same detection method as described above may be employed for the nucleic acid chain (oligonucleotide chain) moiety thereof.

### (11) In the steps of detecting the label in (1)-(10) above, known treatment approaches may be performed in combination.

Examples of such treatment approaches, according to a conventional western blotting, include an approach of identifying and quantifying using a scanner, and an approach of detecting, separating and collecting cells or compounds containing the antigen of interest using a flow cytometry, a cell sorter or an appropriate support.

### 4. Other steps

The present invention may further comprise steps other than the steps described above and is not limited thereto. These other steps may be carried out according to known means and methods.

### 5. Kit for identification and detection

A kit for identifying and detecting target substances according to the present invention comprises, as components, multiple types of binders that are labeled so as to be identified according to the types of the target substances. For details of the binders, the description of the detection method of the present invention above (specifically, description of "2.(3)" above) similarly applies.

The kit of the present invention can be used effectively for performing the detection method of the present invention described above and thus is very useful.

The kit of the present invention may comprise, components other than the components above. Examples of other components include restriction enzymes, DNA polymerases, PCR primers, dNTPs, various buffers, sterile water, Eppendorf tubes, phenol chloroform, chloroform, ethanol, nucleic acid coprecipitating agents, various gels (powder), free radical-generating/liberating reagents (HRP, Fe complex, etc.), serum components as blocking agents such as bovine serum albumin (BSA), skim milk and goat serum, various detergents, fluorescent reagents such as DNA intercalator, photoreactive substances, various plates, antiseptics such as sodium azide, an experiment operating manual (instruction) and the like, as well as, if necessary, experimental instruments such as various electrophoresis devices and PCR.

Hereinafter, the present invention will be described more specifically by way of examples. The present invention, however, is not limited to these examples.

### [Example 1]

### <Identification and detection by PCR>

### 1. Preparation of conjugated antibody and assay

### (1) Preparation of oligonucleotide chain

A 101-mer oligonucleotide was prepared by PCR using a primer represented by SEQ ID NO:1 having biotin bound at the 3'terminal as 5'primer (5-MUSTagRIBio) and a primer represented by SEQ ID NO:2 having FAM bound at the 5' terminal end as 3'primer (3-MUSTag101FAM).

A 203-mer oligonucleotide was prepared by PCR using a primer represented by SEQ ID NO:1 having biotin bound at the 3'terminal as 5'primer (5-MUSTagRIBio) and a primer represented by SEQ ID NO:3 having FAM bound at the 5' terminal end as 3'primer (3-MUSTag203FAM).
5-MUSTagRIBio:
Biotin-CGGAATTCGCGGACGAGGAGAAGCTGCCGCCCGGCTGG (SEQ ID NO: 1)
3-MUSTag101FAM:
FAM-GCTGGCGTTAGTGATGTGGTTGAA (SEQ ID NO:2)
3-MUSTag203FAM:
FAM-CTGGCTGTGCTTCACCAG (SEQ ID NO:3)

Each of the PCR described above was performed using Pin1-inserted pcDNA3.1 (Invitrogen) as template DNA and Taq polymerase as polymerase with the following reaction solution composition under the following reaction conditions.

### <<Reaction solution composition>>

| | |
|---|---|
| Template DNA (100 µg/µL): | 1 µL |
| Taq polymerase: | 2.5 unit |
| 5'primer (20 µM): | 2 µL |
| 3'primer (20 µM): | 2 µL |
| dNTP (2.5 mM each): | 8 µL |
| 10×Buffer: | 10 µL |
| Sterile water: | Suitable amount (about 77µL) |
| Total: | 100 µL |

### «Reaction conditions»

Total of 30 cycles of the following cycle conditions: "heat denaturation/dissociation for 1 minute at 95°C; annealing for 1 minute at 55°C; synthesis/elongation for 30 seconds at 72°C".

The amplified products after each of the PCR described above are purified into single oligonucleotides by subjecting supernatants centrifuged after PCR to filter purification with a MinElute PCR Purification spin column (Qiagen) or a Miniprepcolumn (Invitrogen).

### (2) Preparation of biotinylated antibodies

According to a conventional method, the following two types of monoclonal antibodies were produced.
12CA5 (anti-HA monoclonal antibody)
9E10 (anti-Myc monoclonal antibody)

Then, 12CA5 and 9E10 antibodies were each mixed with Sulfo-NHS-LC-Biotin (Pierce) at a molar ratio of 1:20 and allowed to react for 30 minutes at room temperature. Each of the reaction solutions was passed through a 5 mL desalinating column and an antibody fraction of interest was collected to obtain two types of biotinylated antibodies.

### (3) Preparation of oligonucleotide-conjugated antibodies

The 101- and 203-mer oligonucleotides were mixed with biotinylated 12CA5 and biotinylated 9E10, respectively at a molar ratio of 1:1. Then, to each of them, NeutrAvidin (PIERCE Biotechnology) was added at a molar ratio to the biotinylated antibody of 1:1, and allowed to react for 15 minutes at room temperature. Each of these reaction solutions was passed through a 5 mL desalinating column and a fraction of interest was collected to obtain two types of oligonucleotide-conjugated antibodies.

### (4) Assay for antigen specificity and identification/detection capacity

For the obtained two types of conjugated antibodies, their antigen specificities (antigen-antibody reactivity) and identification/detection capacities for each label were confirmed as follows.

First, HA and Myc synthetic peptides were prepared as antigens, and plastic 96-well plates were coated with each of these synthetic peptides according to a conventional ELISA method. Then, the plates were blocked with 5% milk in PBST solution overnight and washed thoroughly with PBS. Subsequently, to the wells of each plate, the previously prepared solutions containing the two types of oligonucleotide-conjugated antibodies were added at a suitable amount, and allowed to react for an hour at room temperature while shaking.

Following reaction, each plate was washed with PBS for three times, then an EcoRI enzyme solution prepared with an EcoRI buffer solution was added to the wells and reacted for 2 hours at 37°C to cleave the oligonucleotide chains in the oligonucleotide-conjugated antibodies.

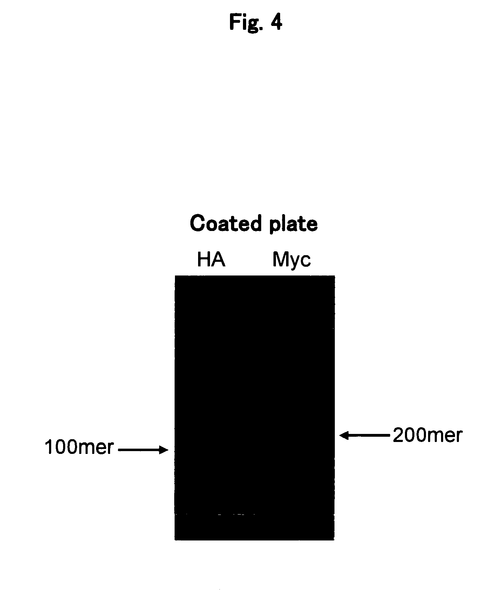

Subsequently, the resultants were collected, if necessary after concentrating with a spin column, and subjected to 1% agarose gel electrophoresis. Bands detected after the electrophoresis are shown in Figure 4.

As a result, the oligonucleotide fragment collected from the HA-coated plate was confirmed to be about 100 mers while the oligonucleotide fragment collected from the Myc-coated plate was about 200 mers.

Thus, through identification and detection by difference in the lengths of the oligonucleotide fragments after the restriction enzyme treatment, it can be confirmed that only the conjugated antibody of 12CA5 and the 101-mer oligonucleotide reacted specifically with HA while only the conjugated antibody of 9E10 and the 203-mer oligonucleotide reacted specifically with Myc.

### 2. Identification and detection using conjugated antibody

The prepared two types of conjugated antibodies were used for performing "antigen-antibody reaction" and "identification and detection for each label" as described below.

According to a conventional antigen-antibody reaction sandwich technique, antibody-bound beads were obtained by binding 9E10 (anti-Myc monoclonal antibody) or 12CA5 (anti-HA monoclonal antibody) to magnetic beads (BioLabs) with a particle size of 1 µm as a support. As an antigen, originally prepared HA-GST-Myc synthetic peptide (antigen solution) was used. First, 9E10-bound beads and 12CA5-bound beads were mixed at a quantitative ratio (particle count ratio) of 3:1, 1:1, 1:3 in three microtubes, to which an antigen solution was added, and incubated for 30 minutes at room temperature. These were washed thoroughly with PBST for three times. To each of them, a suitable amount of solution containing the two types of oligonucleotide-conjugated antibodies was added to react for 30 minutes at room temperature while shaking. Subsequently, they were further washed thoroughly with PBST for three times. To the centrifuged residue, the primer represented by SEQ ID NO:1 (5-MUSTagRIBio) and the primer represented by SEQ ID NO:2 (3-MUSTag 101FAM) as well as the primer represented by SEQ ID NO:1 (5-MUSTagRIBio) and the primer represented by SEQ ID NO:3 (3-MUSTag203FAM) were added (in other words, the primers represented by SEQ ID NOS: 1, 2 and 3 were added) to perform PCR with the following reaction solution composition under the following reaction conditions.

### <<Reaction solution composition>>

| Template DNA (centrifuged residue): | Amount of residue in each tube |
|---|---|
| Taq polymerase: 5' primer | 2.5 unit |
| 5-MUSTagRIBio (20 µM): 3'primers | 4 µL |
| 3-MUSTag101FAM (20 µM): | 2 µL |
| 3-MUSTag203FAM (20 µM): | 2 µL |
| dNTP (2.5mM each): | 8 µL |
| 10×Buffer: | 10 µL |
| Sterile water: | Suitable amount (about 70 µL) |
| Total: | 100 µL |

### «Reaction conditions»

Total of 5 cycles of the following cycle conditions: "heat denaturation/dissociation for 30 seconds at 94°C; annealing for 30 seconds at 60°C; synthesis/elongation for 30 seconds at 72°C".

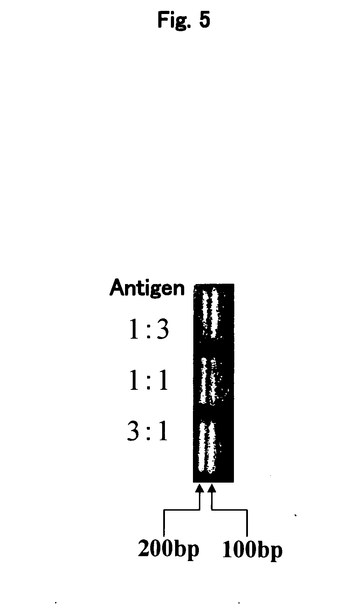

Supernatants obtained by centrifugation after PCR was subjected to 1% agarose gel electrophoresis. Bands detected after the electrophoresis are shown in Figure 5.

As a result, in the samples of all of the quantitative ratios, bands representing about 100 bp and about 200 bp fragments were observed, confirming that each of the oligonucleotide labels can be identified and detected in the same reaction vessel. Moreover, the amounts of the fragments corresponded to the quantitative ratio of the antibody-bound beads, confirming that identification and detection are also possible in a quantitative ratio dependent manner (quantitatively).

### [Example 2]

### <Identification and detection using micro fluid analyzer>

### 1. Preparation of conjugated antibody

### (1) Preparation of oligonucleotide chain

A 300-mer oligonucleotide was prepared by PCR using a primer represented by SEQ ID NO:4 having biotin bound at the 3'terminal as 5'primer
(5-pcDNA3Bio840) and a primer represented by SEQ ID NO:5 having FAM bound at the 5' terminal end as 3'primer (3-MUSTag1140FAM).

A 500-mer oligonucleotide was prepared by PCR using a primer represented by SEQ ID NO:4 (5-pcDNA3Bio840) having biotin bound at the 3'terminal as 5'primer and a primer represented by SEQ ID NO:6 (3-MUSTag1340FAM) having FAM bound at the 5' terminal end as 3'primer.
5-pcDNA3Bio840:
Biotin-CTTACTGGCTTATCGAAA (SEQ ID NO:4)
3-MUSTag1140FAM:
FAM-CATTTTATTAGGAAAGGA (SEQ ID NO:5)
3-MUSTag1340FAM:
FAM-CGCGCTTAATGCGCCGCT (SEQ ID NO:6)

Each of the PCR above was performed using pcDNA3.1 (Invitrogen) as template DNA and Taq polymerase as polymerase with the following reaction solution composition under the following reaction conditions.

### <<Reaction solution composition>>

| | |
|---|---|
| Template DNA (100 µg/µL): | 1 µL |
| Taq polymerase: | 2.5 unit |
| 5'primer (20 µM): | 2 µL |
| 3'primer (20 µM): | 2 µL |
| dNTP (2.5 mM each): | 8 µL |
| 10 × Buffer: | 10 µL |
| Sterile water: | Suitable amount (about 77 µL) |
| Total: | 100 µL |

### <<Reaction conditions>>

Total of 30 cycles of the following cycle conditions: "heat denaturation/dissociation for 1 minute at 95°C; annealing for 1 minute at 55°C; synthesis/elongation for 30 seconds at 72°C".

The amplified products after each of the PCR described above were purified into single oligonucleotides by subjecting supernatants obtained by centrifugation after PCR to filter purification with a MinElute PCR Purification spin column (Qiagen).

### (2) Preparation of biotinylated antibodies

Biotinylated 12CA5 and biotinylated 9E10 were prepared in the same manner as "1. (2)" of Example 1.

### (3) Preparation of oligonucleotide-conjugated antibodyies

The 300- and 500-mer oligonucleotides were mixed with biotinylated 12CA5 and biotinylated 9E10, respectively at a molar ratio of 1:1. Then, to each of them, NeutrAvidin (PIERCE Biotechnology) was added at a molar ratio to the biotinylated antibody of 1:1, and allowed to react for 15 minutes at room temperature. Each of these reaction solutions was passed through a 5 mL desalinating column and a fraction of interest was collected to obtain two types of oligonucleotide-conjugated antibodies. Each oligonucleotide-conjugated antibody was fractionated using a gel filtration column (PC12: Pharmacia) to obtain only the oligonucleotide-conjugated antibodies with 280 nm and 210 nm simultaneous detection systems.

### 2. Identification and detection using conjugated antibodies

The prepared two types of conjugated antibodies were used for performing "antigen-antibody reaction" and "identification and detection for each label" as described below.

According to a conventional antigen-antibody reaction sandwich technique, antibody-bound beads were obtained by binding 12CA5 or 9E10 using magnetic beads (BioLabs) with a particle size of 1 µm as a support. As an antigen, HA-GST-Myc synthetic peptide was used. First, the 12CA5-bound beads or the 9E10-bound beads were mixed with 1, 0.1 or 0.01 pmol/µL HA-GST-Myc synthetic peptide in microtubes and incubated for 30 minutes at room temperature. These were washed thoroughly with PBST for three times. To each of them, a suitable amount of solution containing the two types of oligonucleotide-conjugated antibodies (300- and 500-mer oligonucleotides were liberated by EcoRI treatment) were added and allowed to react for 30 minutes at room temperature while shaking. Subsequently, a suitable amount of EcoRI solution was added to each of the microtubes for reaction for 15 minutes at 37°C to cleave the oligonucleotide chains in the oligonucleotide-conjugated antibodies. The supernatants obtained after centrifugation were analyzed with cosmo-i SV1210 (Hitachi, Ltd.).

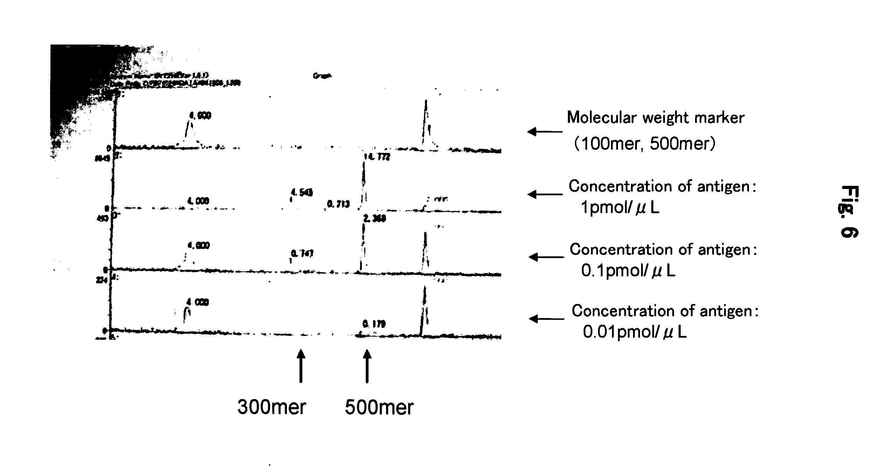

As a result, peaks representing the presence of the 300- and 500-mer oligonucleotide fragments obtained by EcoRI treatment were both confirmed in an antigen concentration dependent manner (see Figure 6).

Also, when a commercially available nucleic acids analyzer was used, the presence of both 300- and 500-mer oligonucleotide fragments were found to be detected in an antigen concentration dependent manner (quantitatively).

### [Example 3]

### <Identification and detection by PCR >

### 1. Preparation of conjugated antibody using adapter

According to the following procedure, a conjugated antibody was prepared in which an oligonucleotide chain was bound to an antibody molecule via "protein G" as an adapter moiety.

### (1) Preparation of oligonucleotide chain

A 100-mer oligonucleotide was prepared by PCR using a primer represented by SEQ ID NO:7 having biotin bound at the 5' terminal end as 5'primer (5-pcDNA3Bio840) and a primer represented by SEQ ID NO:8 as 3' primer (3-pGEX+MUST100).

A 200-mer oligonucleotide was prepared by PCR using a primer represented by SEQ ID NO:7 having biotin bound at the 5' terminal end as 5'primer (5-pcDNA3Bio840) and a primer represented by SEQ ID NO:9 as 3'primer (3-pGEX+MUST200).

A 300-mer oligonucleotide was prepared by PCR using a primer represented by SEQ ID NO: 7 having biotin bound at the 5' terminal end as 5'primer (5-pcDNA3Bio840) and a primer represented by SEQ ID NO:10 as 3'primer (3-pGEX+MUST300).

A 400-mer oligonucleotide was prepared by PCR using a primer represented by SEQ ID NO:7 having biotin bound at the 5' terminal end as 5'primer (5-pcDNA3Bio840) and a primer represented by SEQ ID NO:11 as 3'primer (3-pGEX+MLTST400).

A 500-mer oligonucleotide was prepared by PCR using a primer represented by SEQ ID NO:7 having biotin bound at the 5' terminal end as 5'primer (5-pcDNA3Bio840) and a primer represented by SEQ ID NO: 12 as 3'primer (3-pGEX+MUST500).
5-pcDNA3Bio840:
Biotin-CTTACTGGCTTATCGAAA (SEQ ID NO:7)
3-pGEX+MUST100:
GGCAAGCCACGTTTGGTG CAGTCGAGGC TGATCAGCGA (SEQ ID NO: 8)
3-pGEX+MUST200:
GGCAAGCCACGTTTGGTG TCCTCATTTT ATTAGGAAAG (SEQ ID NO: 9)
3-pGEX+MUST300:
GGCAAGCCACGTTTGGTG AGCATGCCTG CTATTGTCTT (SEQ ID NO:10)
3-pGEX+MUST400:
GGCAAGCCACGTTTGGTG CCCGCCGCGC TTAATGCGCC (SEQ ID NO: 11)
3-pGEX+MUST500:
GGCAAGCCACGTTTGGTG AAAGCCGGCG AACGTGGCGA (SEQ ID NO:12)

Each of the PCR above was performed using pGEX (Pharmacia) as template DNA and Taq polymerase as polymerase with the following reaction solution composition under the following reaction conditions.

### <<Reaction solution composition>>

| | |
|---|---|
| Template DNA (100 µg/µL): | 1 µL |
| Taq polymerase: | 2.5 unit |
| 5'primer (20 µM): | 2 µL |
| 3'primer (20 µM): | 2 µL |
| dNTP (2.5 mM each): | 8 µL |
| 10×Buffer: | 10 µL |
| Sterile water: | Suitable amount (about 77 µL) |
| Total: | 100 µL |

### <<Reaction conditions>>

Total of 30 cycles of the following cycle conditions: "heat denaturation/dissociation for 1 minute at 95°C; annealing for 1 minute at 55°C; synthesis/elongation for 30 seconds at 72°C".

The amplified products after each of the PCR described above are purified into single oligonucleotides by subjecting supernatants obtained by centrifugation after PCR to filter purification with a MinElute PCR Purification spin column (Qiagen).

### (2) Preparation of protein G/avidin binding unit

### (i) Preparation of maleimide-activated protein G

Five mg of protein G was dissolved in 500 µL 50 mM sodium phosphate (pH 7.4). Then, 1 mg of sulfosuccinimidyl 4- (N-maleimidomethyl) cyclohexane-1-carboxylate (hereinafter, "Sulfo-SMCC" (PIERCE 50mg, # 22322, Molecular Weight: 436.37, Spacer Arm Length: 11.6 Å; see structural formula (I) below)) as a chemical modifier (chemical linker) was dissolved in 50 µL DMSO, and added to the solution of protein G above.

The solution following addition was slowly agitated for 60 minutes at room temperature to allow reaction and binding between protein G and Sulfo-SMCC. This binding was established between Sulfo-NHS ester group of Sulfo-SMCC and protein G.

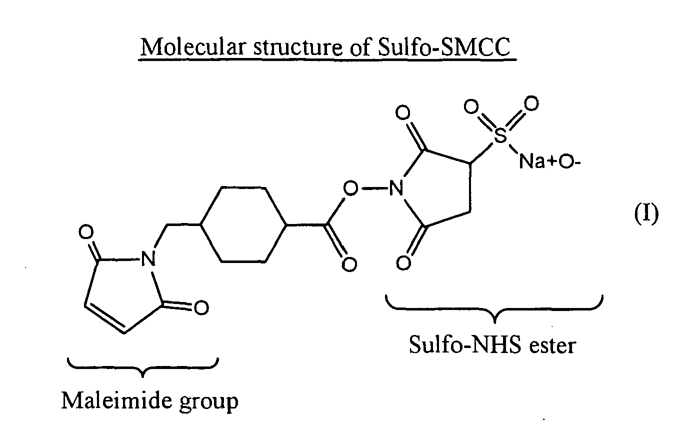

In order to remove excessive Sulfo-SMCC remaining after the reaction, a desalinating columns equilibrated with a binding buffer were used to fractionate the reaction solution into 500 µL each. The concentration of the eluted protein (maleimide-activated protein G) in each fraction was measured to collect the fraction at peak.

A 5 kDa pore size filter tube was used to concentrate maleimide-activated protein G by centrifugation to adjust the final volume of the solution to 500 µL.

### (ii) Binding with avidin

EZ-Link maleimide-activated NeutroAvidin (PIERCE 5mg, #31007, 60 kDa) was dissolved in a buffer (100 mM Na phosphate (pH 7.6), 5mM EDTA). To this solution, a maleimide-activated protein G solution was added. This addition was performed according to the following quantitative ratio such that the molar ratio of protein G and avidin (protein G : avidin) was about 1:3.
Protein G (22kDa) : Avidin (60kDa) = 1 mg : 1 mg

The mixed solution was slowly agitated for reaction for at least 4 hours (up to overnight) at 4°C.

The reacted solution was applied on a gel filtration column (FPLC gel filtration (PC12 column)) to collect, from the protein G/avidin binding unit, a fraction of a molecular weight corresponding to the binding unit in which 2 or 3 molecules of avidin were bound to a molecule of protein G.

### (3) Preparation of conjugated antibody

### (i) Binding between protein G/avidin binding unit and oligonucleotide chain

Each of the purified oligonucleotide chains (biotinylated oligo) obtained in (1) above and the protein G/avidin binding unit obtained in (2) above were mixed in a buffer (50 mM Tris (pH 8.0), 1 mM EDTA, 0.1 M NaCl, 0.5% gelatin, 0.1% Tween20) at a molar ratio (biotinylated oligo: protein G/avidin binding unit) of about 1:2. The mixed solution was agitated for 30 minutes at room temperature for reaction (biotin-avidin binding reaction) to obtain an oligonucleotide/protein G binding unit.

### (ii) Preparation of antibodies

The following five types of monoclonal antibodies were prepared according to a conventional method.
Anti-Int6 antibody (Anti-mouse IgG monoclonal antibody)
Anti-HIF 1α-antibody (Anti-rabbit IgG monoclonal antibody)
12CA5 (anti-HA monoclonal antibody)
9E10 (anti-Myc monoclonal antibody)
Anti-beta-galactosidase antibody (Anti-beta-galactosidase monoclonal antibody)

### (iii) Binding between antibody and adapter moiety

The oligonucleotide/protein G binding unit and each types of monoclonal antibodies were mixed in a buffer (50 mM Tris (pH 8.0), 1mM EDTA, 0.1M NaCl, 0.5% gelatin, 0.1% Tween20) at a molar ratio of 1:1, and agitated for reaction for an hour at room temperature or for 12 hours at 4°C. Specifically, anti-Int6 antibody (anti-mouse IgG monoclonal antibody) was mixed with the 100-mer oligonucleotide-bound binding unit, anti-HIF1α antibody (anti-rabbit IgG monoclonal antibody) was mixed with the 200-mer oligonucleotide-bound binding unit, 12CA5 was mixed with the 300-mer oligonucleotide-bound binding unit, 9E10 was mixed with the 400-mer oligonucleotide-bound binding unit and anti-beta-galactosidase antibody (anti-beta-galactosidase monoclonal antibody) was mixed with the 500-mer oligonucleotide-bound binding unit for reaction. Each of the reaction solutions was passed through a 5 mL desalinating column to collect the fractions of interest, resulting in five types of oligonucleotide-conjugated antibodies were obtained.

### 2. Identification and detection using conjugated antibodies

The prepared five types of conjugated antibodies were used to perform "antigen-antibody reaction" and "identification and detection for each label" as described below.

According to a conventional ELISA method, on the wells of ELISA plate, five types of antigens (mouse IgG, rabbit IgG, HA, Myc and beta-galactosidase (50ng each)) were immobilized. Blocking was performed by adding 5% skim milk 100 mM Na phosphate 150 mM NaCl (pH 7.4) to the wells and left for 30 minutes at room temperature. To these wells, 20 µL each of the solutions containing five types of oligonucleotide-conjugated antibodies was added and reacted for 30 minutes at room temperature while shaking. After the reaction, the resultant was washed thoroughly with 0.1% NP40, 100 mM Na phosphate 150 mM NaCl (pH 7.4) for five times, and then washed once with 100 mM Na phosphate 150 mM NaCl (pH 7.4). A suitable amount ofEcoRI solution (10 unit/20 µL) was added to each well and reacted for 30 minutes at 37°C. to liberate the oligonucleotide chains of the conjugated antibodies bound to the antigens. Supernatants containing these free oligonucleotides were collected.

Subsequently, to these supernatants (i.e., using free oligonucleotides as a template), a primer represented by SEQ ID NO:13 (5-Forw-3) and a primer represented by SEQ ID NO:14 fluorescently labeled with FAM dye (3-pGEX-FAM) were added to perform PCR with the following reaction solution composition under the following reaction conditions.
5-Forw-3: TGCATCTAGAGGGCCCTATTCTATA (SEQ ID NO:13)
3-pGEX-FAM: FAM-GGCAAGCCACGTTTGGTG (SEQ ID NO:14)

### <<Reaction solution composition>>

| | |
|---|---|
| EcoRI-treated supernatant: | 5 µL |
| Taq polymerase: 5' primer | 2.5 unit |
| 5-Forw-3 (20 mM): 3' primer | 2 µL |
| 3-pGEX-FAM (20 mM): | 2 µL |
| dNTP (2.5mM): | 8 µL |
| 10×Buffer: | 10 µL |
| Sterile water: | Suitable amount (about 73µL) |
| Total: | 100 µL |

### <<Reaction conditions>>

Total of 15 cycles of the following cycle conditions: "heat denaturation/dissociation for 5 minutes at 95°C; annealing for 30 seconds at 55°C; synthesis/elongation for 30 seconds at 72°C". Subsequently, 1 µl of the reaction solution was dissolved in 10 µl formamide and subjected to heat denaturation for 5 minutes at 95°C.

The reaction solution after PCR was centrifuged. The obtained supernatant was analyzed with GeneScan software using DNA sequencer ABI-3100 (Applied Biosystems) to identify position and height of each peak.

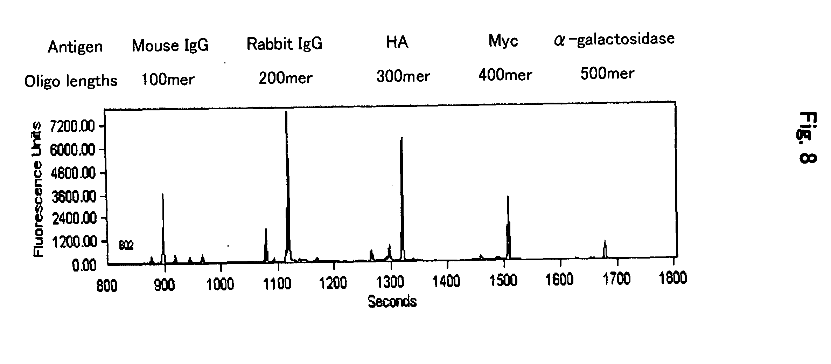

As a result, peaks representing the presence of the oligonucleotide fragments of the conjugated antibodies that can specifically bind to one of the antigens (mouse IgG, rabbit IgG, HA, Myc and beta-galactosidase), i.e., oligonucleotide fragments with oligo lengths of 100 mers, 200 mers, 300 mers, 400 mers and 500 mers, were confirmed (see Figure 8).

### [Reference Example 1]

### <Comparison of detection sensitivities between the detection method of the present invention and conventional ELISA method >

### 1. Preparation of primary antibody used in ELISA method

According to a convention method, mouse anti-HA antibody and mouse anti-Myc antibody were prepared.

### 2. Preparation of oligonucleotide-conjugated antibody used in the detection method of the present invention

According to the same method as "1." of Example 3, a "12CA5-conjugated antibody having a 200-mer oligonucleotide chain" (hereinafter, conjugated antibody A) was prepared.

Specifically, the conjugated antibody A above was prepared in the same manner as "1." of Example 3 for the method for preparing the "conjugated antibody having a 100-mer oligonucleotide chain" among the five types of the oligonucleotide-conjugated antibodies used in Example 3 except that an oligonucleotide/protein G binding unit having a 200-mer oligonucleotide chain was reacted with anti-Int6 antibody instead of reacting an oligonucleotide/protein G binding unit having a 100-mer oligonucleotide chain with anti-Int6 antibody (anti-mouse IgG monoclonal antibody).

### 3. Immobilization of antigen

HA-GST-Myc synthetic peptide was used as an antigen. According to a conventional ELISA method, the antigen was immobilized on each well of the ELISA plate at an amount indicated in the table below.

Blocking was performed by adding 5% skim milk, 100 mM Na phosphate, 150 mM NaCl (pH 7.4) to the wells and leaving for 30 minutes at room temperature.

**Antigen amount in each well**

| | A | B | C | D | E | F | G | H |
|---|---|---|---|---|---|---|---|---|
| Column 02 Column 04 | 1.3 pg | 256 fg | 51 fg | 10.2 fg | 2.0 fg | 410 ag | 82 ag | 1.6 ag |
| Column 01 Column 03 | 500 ng | 100 ng | 20 ng | 4 ng | 800 pg | 160 pg | 32 pg | 6.4 pg |

### 4. Detection by ELISA

Following the blocking in 3. above, the resultant was washed thoroughly with 0.1% NP40 and phosphate buffer saline (pH 7.4) for three times.

As primary antibodies, the mouse anti-HA antibody prepared in 1. above was added to each of the wells along columns 01 and 02 of the plate and mouse anti-Myc antibody to each of the wells along columns 03 and 04 of the plate.

The resultant was washed thoroughly with 0.1% NP40, phosphate buffer saline (pH 7.4) for three times and added with HRP-labeled goat anti-mouse IgG.

The resultant was washed thoroughly with 0.1% NP40, phosphate buffer saline (pH 7.4) for three times, added with a substrate and left to stand. Thereafter, color development was terminated with 1% SDS.

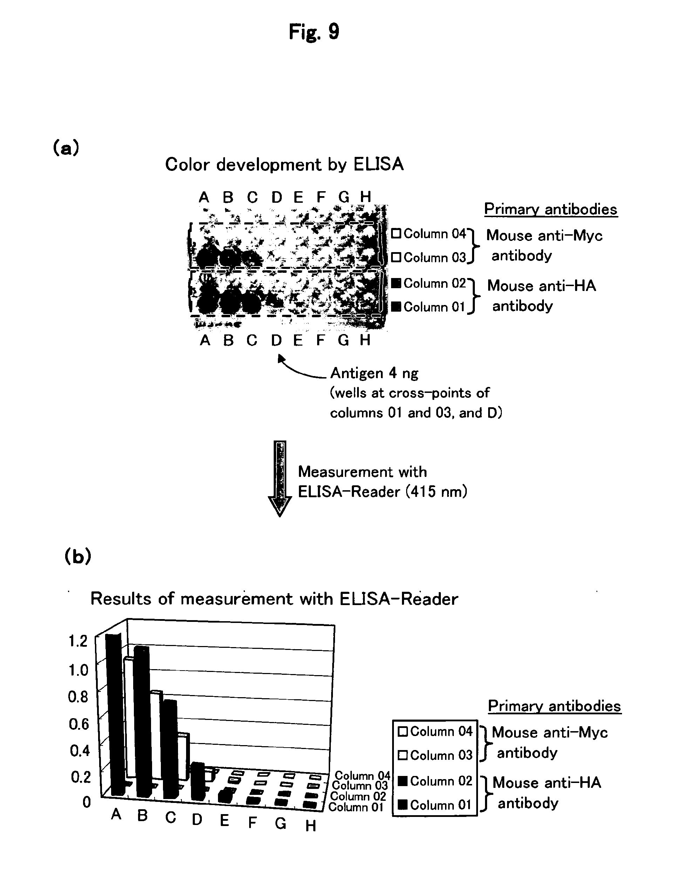

For each of the wells of the plate after color development (Figure 9(a)), the results of measurements using an ELISA-Reader (415nm) are shown in Figure 9(b). From these results, the maximum sensitivity in the ELISA method was confirmed with an antigen amount of 4ng (well at the cross-point of column 01 and D).

### 5. Detection by the method of the present invention

After the blocking of 3. above, the wells were washed thoroughly with 0.1% NP40, 100 mM Na phosphate and 150 mM NaCl (pH 7.4) for three times.

As primary antibodies, the mouse anti-HA antibody prepared in 1. above was added to each of the wells along columns 01 and 02 of the plate and mouse anti-Myc antibody to each of the wells along columns 03 and 04 of the plate.

The resultant was washed thoroughly with 0.1% NP40 and phosphate buffer saline (pH 7.4) for three times. Then, 20 µL of a solution containing the conjugated antibody A prepared in 2. above was added to each of the wells along columns 01 and 02 of the plate and allowed to react for 30 minutes at room temperature while shaking.

Following the reaction, the wells were washed thoroughly with 0.1% NP40, 100 mM Na phosphate and 150 mM NaCl (pH 7.4) for three times and then once by 100 mM Na phosphate and 150 mM NaCl (pH 7.4). A suitable amount of EcoRI solution (10 unit/20 µL) was added to the wells and allowed to react for 30 minutes at 37°C to liberate the oligonucleotide chain of the conjugated antibody A bound to the antigen. Supernatants containing this free oligonucleotide were collected.

Subsequently, to this supernatant (i.e., using the free oligonucleotide as a template), a primer represented by SEQ ID NO: 13 (5-Forw-3) and a primer represented by SEQ ID NO: 14 fluorescently labeled with FAM dye (3-pGEX-FAM) were added, and the resultant was subjected to PCR using the same reaction solution composition under the same reaction conditions as "2." of Example 3 (except the number of cycles was 20).
5-Forw-3: TGCATCTAGAGGGCCCTATTCTATA (SEQ ID NO:13)
3-pGEX-FAM: FAM-GGCAAGCCACGTTTGGTG (SEQ ID NO: 14)

The reaction solution after PCR was centrifuged, and the obtained supernatant was subjected to analysis by GeneScan software using DNA sequencer ABI-3100 (Applied Biosystems) to detect the peaks (positions and heights of the peaks).

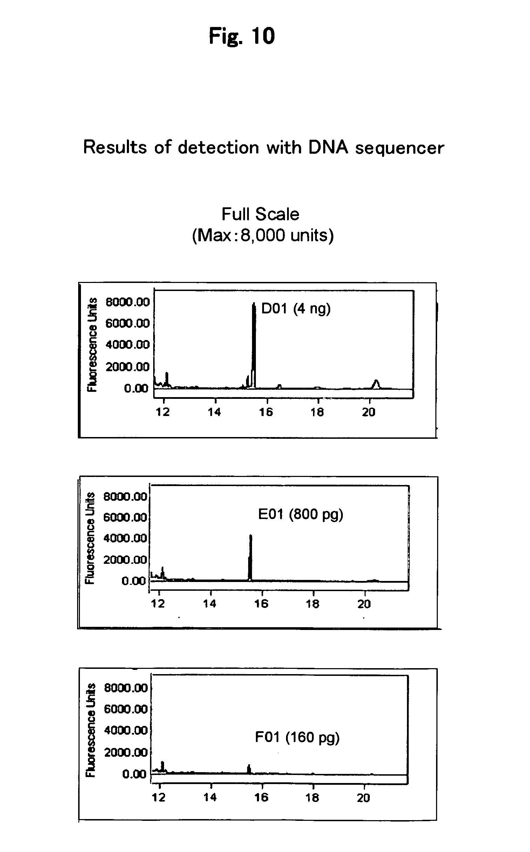

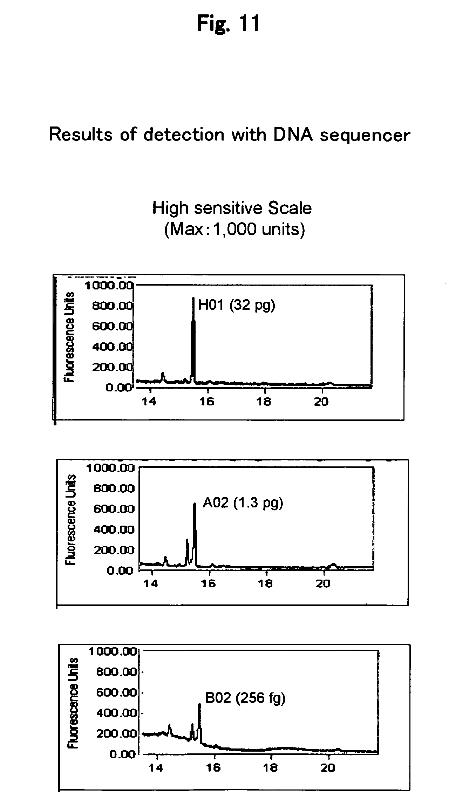

As a result, as shown in Figures 10 and 11, according to the detection method of the present invention, an antigen amount as low as at least 256 fg (well at the cross-point of column 02 and B) was found to be detected effectively. Thus, the detection sensitivity of the detection method of the present invention (PCR: 20 cycles) was confirmed to be higher by 15,000 times compared to that of an ELISA method whose maximum sensitivity was 4 ng.

### [Reference Example 2]

### <Quantitative characteristics of the detection method of the present invention>

### 1. Preparation of oligonucleotide-conjugated antibody

Employing the same method as "1." of Example 3, a "9E10-conjugated antibody having a 100-mer oligonucleotide chain" (hereinafter, conjugated antibody B) was prepared.

Specifically, the conjugated antibody B above was prepared in the same manner as "1." of Example 3 for the method for preparing the "conjugated antibody having a 400-mer oligonucleotide chain" among the five types of oligonucleotide-conjugated antibodies used in Example 3, except that an oligonucleotide/protein G binding unit having a 100-mer oligonucleotide chain was reacted with 9E10 instead of reacting an oligonucleotide/protein G binding unit having a 400-mer oligonucleotide chain with 9E10 (anti-Myc monoclonal antibody).

### 2. Immobilization of antigen

According to a conventional ELISA method, Myc peptide as an antigen was immobilized on each well of the ELISA plate at different amounts. Specifically, 50 ng, 12.5 ng, 3.1 ng, 780 pg, 190 pg, 48.8 pg, 12.2 pg and 3 pg of Myc were immobilized on respective wells. An antigen other than Myc (see table below) was also immobilized on each well.

| Antigen | Antigen amount/Well |
|---|---|
| HA peptide | 1-5 ng |
| Myc-GST-HA | 1-5 ng |
| Int6 peptide | 1-5 ng |
| HIF peptide | 1-5 ng |
| Beta-galactosidase enzyme protein | 1-5 ng |

Blocking was performed by adding 5% skim milk, 100 mM Na phosphate and 150 mM NaCl (pH 7.4) to each well and left to stand for 30 minutes at room temperature.

### 3. Detection by the method of the present invention

Following blocking, washing was performed for three times with 0.1% NP40, 100 mM Na phosphate and 150 mM NaCl (pH 7.4).

The solution containing the conjugated antibody B prepared in 1. above was added to each well for 20 µL and allowed to react for 30 minutes at room temperature.

After the reaction, the plate was washed thoroughly with 0.1%NP40, 100 mM Na phosphate and 150 mM NaCl (pH 7.4) for three times, and then once with 100 mM Na phosphate and 150 mM NaCl (pH 7.4). Then, and a suitable amount of EcoRI solution (10 unit/20 µL) was added to each well for reaction for 30 minutes at 37°C to liberate the oligonucleotide chain of the conjugated antibody B bound to the antigen. Supernatants containing this free oligonucleotide were collected.

Subsequently, to this supernatant (i.e., using the free oligonucleotide as a template), a primer represented by SEQ ID NO:13 (5-Forw-3) and a primer represented by SEQ ID NO: 14 that is fluorescently labeled with FAM dye (3-pGEX-FAM) were added, and the resultant was subjected to PCR using the same reaction solution composition under the same reaction conditions as "2." of Example 3 (except the number of cycles was 20).
5-Forw-3: TGCATCTAGAGGGCCCTATTCTATA (SEQ ID NO:13)
3-pGEX-FAM: FAM-GGCAAGCCACGTTTGGTG (SEQ ID NO:14)

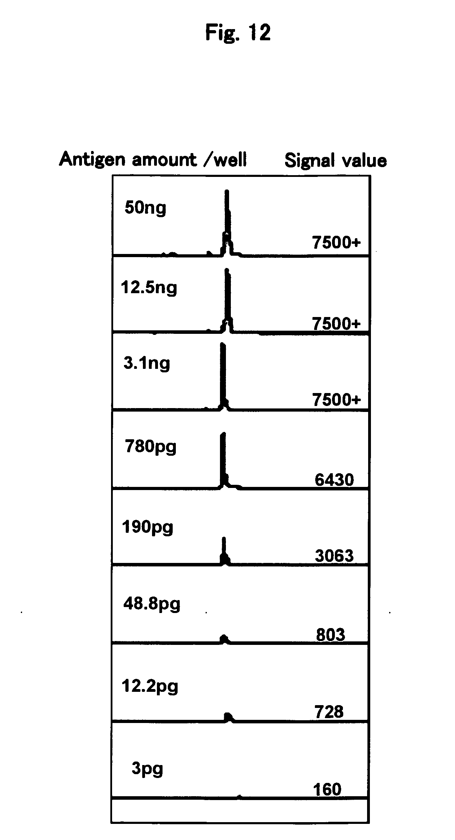

The reaction solution after PCR was centrifuged. The obtained supernatant was subjected to analysis by GeneScan software using DNA sequencer ABI-3100 (Applied Biosystems) to detect the peaks (to identify positions and heights of the peaks). The results are shown in Figure 12.

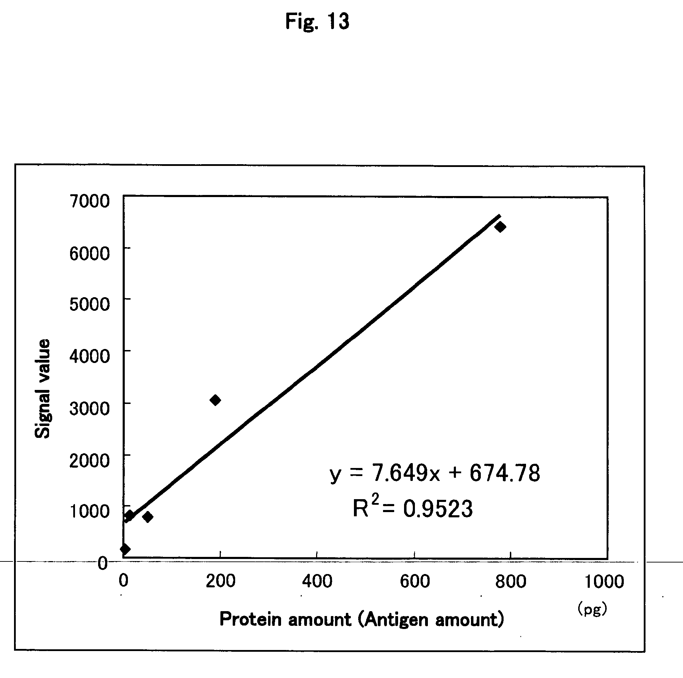

For the obtained detection results, values were plotted with respect to the horizontal axis representing the amount of protein used and the vertical axis representing a signal value (antigen amount: Myc amount) (Figure 13). Signal values exceeding the detection limit are not plotted. As a result, all of the values significantly correlated with each other, confirming that, according to the detection method of the present invention, specific antigens can be detected in a quantitative manner regardless of the type or the number of antigens.

### INDUSTRIAL APPLICABILITY

The present invention provides a method for detecting target substances, which allows identification and detection of multiple types of target substances contained in a specimen at once. The method of the present invention is highly useful in that it can remarkably increase examination or diagnosis efficiency, assessment efficiency or the like especially in the field of human clinical examination including diagnosis of cancer (improvement of the recovery rate by early detection) and drug efficacy assessment of anticancer drugs, diagnosis of cerebral stroke and myocardial infarction (reduction of aftereffects by early diagnosis), and specification of the pathogen in an infectious diseases, infectious diseases of livestock and pets, diagnosis of other diseases, and further in the field of food and environment including specification of food contaminants by pesticide residue or bacteria, or specification of environmental pollutants such as dioxin, PCB or the like.
SEQUENCE LISTING FREE TEXT
SEQ ID NO: 1: Primer
SEQ ID NO:2: Primer
SEQ ID NO: 3 Primer
SEQ ID NO:4: Primer
SEQ ID NO:5: Primer
SEQ ID NO:6: Primer
SEQ ID NO:7: Primer
SEQ ID NO:8: Primer
SEQ ID NO:9: Primer
SEQ ID NO:10: Primer
SEQ ID NO:11: Primer
SEQ ID NO:12: Primer
SEQ ID NO:13: Primer
SEQ ID NO:14: Primer

## Claims

1. A method for identifying and detecting multiple types of target substances contained in a specimen, comprising the steps of: contacting the target substances immobilized and/or nonimmobilized on a support with labeled binders capable of identifying corresponding types of target substances so as to form complexes between the target substances and the binders; and detecting the labels in the binders forming the complexes.

2. A method according to Claim 1, wherein the label moiety of the binder is bound to the binder via at least an adapter moiety.

3. A method according to Claim 2, wherein the adapter moiety is at least one selected from the group consisting of protein G, protein A, protein L and a fusion protein of protein A and protein G.

4. A method according to any one of Claims 1 to 3, wherein the binder forms a conjugate with an oligonucleotide chain.

5. A method according to Claim 4, wherein the labeling treatment is at least one treatment on the oligonucleotide chain selected from the group consisting of: a treatment of providing a site cleavable with a restriction enzyme, a treatment of providing a region amplifiable by PCR; a treatment of incorporating a radioisotope element; a treatment of binding a fluorescent dye; a treatment of binding an enzyme; a treatment of providing a site cleavable by light irradiation; a treatment of providing a site cleavable with active oxygen; a treatment of binding a labeled dendrimer; and a treatment of providing a difference by at least one base type.

6. A method according to Claim 5, wherein the step of detecting the label is a step of detecting the PCR-amplified product by electrophoresis.

7. A method according to any one of Claims 1 to 3, wherein the binder forms a conjugate with an oligopeptide chain.

8. A method according to Claim 7, wherein the labeling treatment is at least one treatment on the oligopeptide chain selected from the group consisting of: a treatment of providing a site cleavable with protease; a treatment of incorporating a radioisotope element; a treatment of binding a fluorescent dye; a treatment of binding an enzyme; a treatment of providing a site cleavable by light irradiation; a treatment of providing a site cleavable with active oxygen; and a treatment of binding a labeled dendrimer.

9. A method according to any one of Claims 1 to 8, wherein the target substance is an antigen and the binder is an antibody.

10. A kit for identifying and detecting target substances, comprising multiple types of labeled binders capable of identifying corresponding types of target substances.
